# EUROPEAN PATENT APPLICATION

(11) **EP 3 162 905 A1**
(43) Date of publication of application: **03.05.2017**
(21) Application number: 16200867.6
(22) Date of filing: 21.01.2014
(51) Int. Cl.: C22B 3/22

(54) **METHOD FOR METAL CONCENTRATION, METHOD FOR METAL RECOVERY, DEVICE FOR METAL CONCENTRATION, AND DEVICE FOR METAL RECOVERY**

(30) Priority: 21.01.2013 JP 2013008537
(62) Divisional of application: 14740625.0
(71) Applicant: Mitsubishi Rayon Co. Ltd., Tokyo 100-8253 (JP); Osaka Prefecture University Public Corporation, Osaka 599-8531 (JP)
(72) Inventor: KONISHI, Yasuhiro, Sakai-shi, Osaka 599-8531 (JP); SAITOH, Norizoh, Sakai-shi, Osaka 599-8531 (JP); AMBO, Takanori, Toyohashi-shi,, Aichi 440-8601 (JP); TERAZAWA, Kaoru, Chiyoda-ku, Tokyo 100-8253 (JP); RYUNO, Koichiro, Yokohama-shi,, Kanagawa 230-0053 (JP); NINOMIYA, Yasuhiro, Toyohashi-shi,, Aichi 440-8601 (JP)
(74) Representative: TBK

(57) **Abstract**

An objective of invention is conveniently concentrating a metal in a metal ion-containing solution with high efficiency and to recover the metal from the metal ion-containing solution with high recovery efficiency. A method for concentrating or recovering a metal in a metal ion-containing solution in the present invention is the method comprising the following steps, a reduction and accumulation step to reduce the metal ion into a metal fine particle and also to accumulate the metal fine particle in the microorganism by allowing the microorganism and an electron donor B to act on a metal ion-containing solution W₀ and thus to obtain a solution W₁ that contains a microorganism having a metal fine particle accumulated therein; a concentration step to concentrate the solution W₁ that contains the microorganism having a metal fine particle accumulated therein by a filtration membrane and thus to obtain a concentrated solution W₂; and a return step to return the concentrated solution W₂ to first step above and thus to circulate. Further, A device for metal concentration or recovering in the present invention comprises the following: a storage unit 2 to store the solution W₁, a concentration unit 3 to concentrate the solution W₁ that is transferred from the storage unit 2 by a filtration membrane, and a return unit 4 to return a concentrated solution W₂ that is concentrated in the concentration unit 3 to the storage unit 2.

## Description

### TECHNICAL FIELD

The present invention relates to a method for metal concentration, a method for metal recovery, a device for metal concentration, and a device for metal recovery.

This application is based upon and claims the benefit of priority of the prior Japanese Patent Application No. 2013-008537, filed on January 21, 2013, the entire contents of which are incorporated herein by reference.

### BACKGROUND ART

It has been widely performed to recover a metal from a solution containing a metal such as industrial waste water or pregnant leach liquor for the purpose of reuse of resources other than the purpose to meet the water quality standards of the legal regulation. However, metal recovery from a metal ion-containing solution by a general technique requires high energy consumption and thus environmental impact is great. In particular, metal recovery from a metal ion-containing solution containing a metal to be the target of recovery at a low concentration is not substantially performed for economic reasons. To cope with these problems, there is an efficient method to recover a metal from a metal ion-containing solution using a microorganism. As the method to recover a metal from a metal ion-containing solution using a microorganism, for example, there are the following methods (i) to (iii).
(i) A method in which iron-reducing bacteria such as Shewanella algae are allowed to act on a metal ion-containing solution so as to reduce a metal ion into a metal fine particle by the iron-reducing bacteria and also to accumulate the metal fine particle in the iron-reducing bacteria or to directly adsorb a metal ion on the iron-reducing bacteria, and the iron-reducing bacteria having the metal fine particle accumulated therein or the metal ion adsorbed thereon are recovered, thereby recovering a metal (for example, Patent Documents 1 and 2).
(ii) A method in which iron-oxidizing bacteria are allowed to act on a solution containing ferrous ion so as to oxidize the ferrous ion into ferric ion, the pH of the solution is controlled so as to form iron(III) hydroxide, and the solid-liquid separation is conducted, thereby recovering iron (Patent Document 3).
(iii) A method in which bacteria of the genus Shewanella are added to a colloidal silica solution containing metal impurities such as Cu, Zn, Ni and Mg, only the metal impurities are accumulated in the bacteria of the genus Shewanella, and the bacteria of the genus Shewanella having the metal incorporated therein is recovered, thereby separating the impurities from colloidal silica (Patent Document 4).

In the method (i), it is required to increase the contact efficiency between the metal ion and the microorganism by introducing a great amount of microorganism to act on the metal ion in order to increase the recovery efficiency, but the cost increases when a great amount of microorganism is used. Hence, it is difficult to continuously recover the metal from the metal ion-containing solution with high recovery efficiency at low cost. It is required a method capable of increasing the concentration of the microorganism at the time of accumulating the metal fine particle or adsorbing the metal ion in order to increase the recovery efficiency especially in a case in which the metal ion contained in water at a low concentration is reduced into a metal fine particle by the microorganism and also the metal fine particle is accumulated in the iron-reducing bacteria or the metal ion is directly adsorbed on the iron-reducing bacteria. In addition, in the method (ii), the pH control of the solution is complicated and thus the operation is intricate.

In the method (iii), there are a step of binding the microorganism with the metal which takes one or more days, a step of adding a biocide, and a step of controlling the pH by the addition of an alkali, and thus the steps are intricate and take a long period of time. In addition, in the recovery operation as well, strong stirring is required even after obtaining a flock containing bacteria of the genus Shewanella and the recovery percentage of the metal is not that high (in particular, see Examples of Patent Document 4).

### CITATION LIST

### PATENT DOCUMENT

Patent Document 1: JP 2007-113116 A
Patent Document 2: JP 2010-162442 A
Patent Document 3: JP 2005-238181 A
Patent Document 4: JP 2005-298276 A

### SUMMARY OF THE INVENTION

### PROBLEM TO BE SOLVED BY THE INVENTION

As a method to recover a metal from a solution containing a useful metal resource in industry, various methods have been investigated as described above, but these methods have a problem that the recovery efficiency is low and the steps are intricate and thus an increase in cost is caused in order to increase the recovery efficiency. The aim of the invention is to solve such a problem of the related art.

### MEANS FOR SOLVING PROBLEM

The invention provides a method for metal concentration, a method for metal recovery, a device for metal concentration and a device for metal recovery which are able to conveniently concentrate a metal in a metal ion-containing solution with high efficiency and to recover the metal from the metal ion-containing solution with high recovery efficiency.

In other words, the invention has the following aspects.
[1] A method for concentrating a metal in a metal ion-containing solution, the method including the following steps (1) to (3):
   (1) a reduction and accumulation step to reduce the metal ion into a metal fine particle and also to accumulate the metal fine particle in a microorganism by allowing the microorganism and an electron donor to act on a metal ion-containing solution and thus to obtain a solution that contains the microorganism having a metal fine particle accumulated therein;
   (2) a concentration step to concentrate the solution that contains the microorganism having a metal fine particle accumulated therein by a filtration membrane and thus to obtain a concentrated solution; and
   (3) a return step to return the concentrated solution to step (1) above and thus to circulate.
[2] The method for metal concentration according to [1], in which the metal ion is ions of one or more elements selected from the group consisting of Au, Ag, Pt, Pd, Rh, Ir, Ru and Os.
[3] The method for metal concentration according to [1] or [2], in which a metal that is produced by reducing the metal ion coexists when a microorganism and an electron donor act on a metal ion-containing solution in step (1) above.
[4] The method for metal concentration according to any one of [1] to [3], in which the electron donor is an organic substance having from 1 to 7 carbon atoms.
[5] The method for metal concentration according to any one of [1] to [4], in which the electron donor is at least one or both of an aliphatic carboxylic acid having from 1 to 3 carbon atoms and a salt thereof.
[6] The method for metal concentration according to any one of [1] to [5], in which the electron donor is at least one or both of formic acid and a salt thereof.
[7] The method for metal concentration according to any one of [1] to [6], in which the microorganism is iron-reducing bacteria.
[8] The method for metal concentration according to [7], in which the iron-reducing bacteria are bacteria belonging to the genus Shewanella.
[9] The method for metal concentration according to [8], in which the bacteria belonging to the genus Shewanella are Shewanella algae or Shewanella oneidensis.
[10] The method for metal concentration according to any one of [1] to [9], in which an average pore size of the filtration membrane is from 0.01 to 1.0 µm.
[11] A method for metal recovery, the method for recovering the microorganism that is obtained in the method for metal concentration according to any one of [1] to [10] and has a metal fine particle accumulated therein, the method further including the following step (4):
   (4) a recovery step to recover the solution that contains the microorganism having a metal fine particle accumulated therein so as to have a cell concentration of 1.0 × 10¹⁷ cells/m³ or less.
[12] A method for recovering a metal from a metal ion-containing solution, the method including the following steps (1') to (4'):
   (1') an accumulation step to accumulate a metal ion in a microorganism by allowing a microorganism to act on a metal ion-containing solution and thus to obtain a solution that contains the microorganism having a metal ion accumulated therein;
   (2') a concentration step to concentrate the solution that contains the microorganism having a metal ion accumulated therein by a filtration membrane and thus to obtain a concentrated solution;
   (3') a return step to return the concentrated solution to step (1') above and thus to circulate; and
   (4') a recovery step to recover the solution that contains the microorganism having a metal fine particle accumulated therein so as to have a cell concentration of 1.0 × 10¹⁷ cells/m³ or less.
[13] The method for metal recovery according to [12], in which the metal ion is ions of one or more elements selected from the group consisting of Ga, In, Zn, Sn and a lanthanoid.
[14] The method for metal recovery according to [12] or [13], in which the microorganism is iron-reducing bacteria.
[15] The method for metal recovery according to [14], in which the iron-reducing bacteria are bacteria belonging to the genus Shewanella.
[16] The method for metal recovery according to [15], in which the bacteria belonging to the genus Shewanella are Shewanella algae or Shewanella oneidensis.
[17] The method for metal recovery according to any one of [12] to [16], in which an average pore size of the filtration membrane is from 0.01 to 1.0 µm.
[18] A device for metal concentration, including the following (a) to (c):
   (a) a storage unit to store a solution that is obtained by allowing a microorganism and an electron donor to act on a metal ion-containing solution so as to reduce a metal ion into a metal fine particle and also to accumulate the metal fine particle in the microorganism or by allowing the microorganism to act on a metal ion-containing solution so as to accumulate a metal ion in the microorganism and contains a microorganism having a metal fine particle or a metal ion accumulated therein;
   (b) a concentration unit to concentrate the solution that is transferred from the storage unit and contains the microorganism having a metal fine particle or a metal ion accumulated therein by a filtration membrane; and
   (c) a return unit to return a concentrated solution that is concentrated in the concentration unit to the storage unit.
[19] The device for metal concentration according to [18], the device further including the following (e):
   (e) a measurement unit to measure a cell concentration of the solution that is supplied to the concentration unit and contains the microorganism having a metal fine particle or a metal ion accumulated therein.
[20] A device for metal recovery, the device including the above (a) to (c) and the following (d):
   (d) a recovery unit to recover the solution that contains the microorganism having a metal fine particle or a metal ion accumulated therein from at least one or both of the storage unit and the concentration unit.
[21] The device for metal recovery according to [20], the device further including the following (e):
   (e) a measurement unit to measure a cell concentration of the solution that is supplied to the concentration unit and contains the microorganism having a metal fine particle or a metal ion accumulated therein.

In addition, the invention has the following aspects.
<1> A metal recovery method to recover a specific metal from a solution containing a metal ion, in which:
   a microorganism is allowed to act on the solution containing a metal ion so as to reduce the metal ion into a metal fine particle by the microorganism and also to accumulate the metal fine particle in the microorganism,
   a solution that contains the microorganism having the metal fine particle accumulated therein is transferred to a concentration unit and concentrated by filtering through a filtration membrane, and
   a concentrated solution that contains the microorganism having the metal fine particle accumulated therein is continuously recovered.
<2> A metal recovery method to recover a specific metal from a solution containing a metal ion, in which:
   a microorganism is allowed to act on the solution containing a metal ion so as to directly adsorb the metal ion on the microorganism,
   a solution that contains the microorganism having the metal ion adsorbed thereon is transferred to a concentration unit and concentrated by filtering through a filtration membrane, and
   a concentrated solution that contains the microorganism having the metal ion adsorbed thereon is continuously recovered.
<3> The method for metal recovery according to <1> or <2>, in which the microorganism is iron-reducing bacteria.
<4> The method for metal recovery according to <3>, in which the iron-reducing bacteria are bacteria belonging to the genus Shewanella.
<5> The method for metal recovery according to <4>, in which the bacteria belonging to the genus Shewanella are Shewanella algae or Shewanella oneidensis.
<6> The method for metal recovery according to <1>, in which the metal ion is ions of one or more elements selected from the group consisting of Au, Ag, Pt, Pd, Rh, Ir, Ru and Os.
<7> The method for metal recovery according to <2>, in which the metal ion is ions of one or more elements selected from the group consisting of Ga, In, Zn, Sn and a lanthanoid.
<8> A device for metal recovery, including:
   a concentration unit to which a solution that contains a microorganism obtained by allowing a microorganism to act on a solution containing a metal ion so as to reduce the metal ion into a metal fine particle by the microorganism and also to accumulate the metal fine particle or a microorganism having the metal ion adsorbed thereon is transferred and in which the microorganism having the metal fine particle accumulated therein or the metal ion adsorbed thereon in the solution is concentrated by a filtration membrane and
   a recovery unit in which a concentrated solution obtained by concentrating the microorganism having the metal fine particle accumulated therein or the metal adsorbed thereon is continuously recovered from the concentration unit.
<9> The device for metal recovery according to <8>, the device further includes:
   a reaction tank in which a microorganism is allowed to act on a solution containing a metal ion.
<10> The device for metal recovery according to <8> or <9>, in which the filtration membrane is installed on the circulation line provided to a concentration tank.
<11> The device for metal recovery according to <9> or <10>, in which at least one of the reaction tank and the preceding stage of the reaction tank is provided with a gas removing means to remove oxygen in a solution.

### EFFECT OF THE INVENTION

According to the method for metal concentration or the method for metal recovery of the invention, it is possible to conveniently concentrate a metal in a metal ion-containing solution with high efficiency and thus to recover the metal with high recovery efficiency.

By means of the device for metal concentration or the device for metal recovery according to the invention, it is possible to conveniently concentrate a metal in a metal ion-containing solution with high efficiency and thus to recover the metal with high recovery efficiency.

### BRIEF DESCRIPTION OF DRAWINGS

Fig. 1 is a schematic block diagram illustrating an example of the device for metal recovery of the invention; and

Fig. 2 is a schematic block diagram illustrating an example of the device for metal recovery of the invention.

### MODE(S) FOR CARRYING OUT THE INVENTION

### <Device for metal concentration or device for metal recovery>

The device for metal concentration of the invention has the following (a) to (c):
(a) a storage unit to store a solution that is obtained by allowing a microorganism and an electron donor to act on a metal ion-containing solution so as to reduce a metal ion into a metal fine particle and also to accumulate the metal fine particle in the microorganism or by allowing the microorganism to act on a metal ion-containing solution so as to accumulate a metal ion in the microorganism and contains a microorganism having a metal fine particle or a metal ion accumulated therein;
(b) a concentration unit to concentrate the solution that is transferred from the storage unit and contains the microorganism having a metal fine particle or a metal ion accumulated therein by a filtration membrane; and
(c) a return unit to return a concentrated solution that is concentrated in the concentration unit to the storage unit.

In addition, the device for metal recovery of the invention has the following (d) in addition to (a) to (c) above:
(d) a recovery unit to recover the solution that contains the microorganism having a metal fine particle or a metal ion accumulated therein from at least one or both of the storage unit and the concentration unit.

In the invention, a device which does not include the recovery unit (d) is defined as a device for metal concentration and a device which includes the recovery unit (d) is defined as a device for metal recovery. In other words, a device which includes a recovery unit to take out a solution containing a microorganism to the outside of the system is a device for metal recovery.

The device for metal concentration or device for metal recovery of the invention may include one or both of the following (a₀) and (e):
(a₀) a supply unit to supply the metal ion-containing solution and the microorganism or the metal ion-containing solution, the microorganism and the electron donor to the storage unit; and
(e) a measurement unit to measure a cell concentration of the solution that is supplied to the concentration unit and contains the microorganism having a metal fine particle or a metal accumulated therein.

Hereinafter, the device for metal recovery of the invention will be described with reference to an example thereof.

A device for metal recovery 1 of the present embodiment includes a storage unit 2, a concentration unit 3, a return unit 4, a recovery unit 16, a measurement unit 5 and a supply unit 6.

In the device for metal recovery 1 of this example, the storage unit 2 includes a reaction solution storage tank 10 and a stirring blade 10a. The concentration unit 3 includes a membrane module 14 having a filtration membrane. The reaction solution storage tank 10 of the storage unit 2 and the membrane module 14 of the concentration unit 3 are connected to each other by a pipe 56. The return unit 4 includes a pipe 58 of which one end is connected to the membrane module 14 and the other end is connected to the reaction solution storage tank 10. The reaction solution storage tank 10 of the storage unit 2 and the recovery unit 16 are connected to each other by a pipe 44. The supply unit 6 includes a metal ion-containing solution storage tank 20, a bacterial culture storage tank 22 and an electron donor storage tank 24. The reaction solution storage tank 10 of the storage unit 2 and the metal ion-containing solution storage tank 20 are connected to each other by a pipe 50. The reaction solution storage tank 10 of the storage unit 2 and the bacterial culture storage tank 22 are connected to each other by a pipe 52. The reaction solution storage tank 10 of the storage unit 2 and the electron donor storage tank 24 are connected to each other by a pipe 54.

In addition, the device for metal recovery 1 of this example includes a filtered water storage tank 18, and the membrane module 14 and the filtered water storage tank 18 are connected to each other by a pipe 46 to which a filtration pump 48 is provided in the middle.

### [(a) Storage unit]

To the reaction solution storage tank 10 of the storage unit 2, a metal ion-containing solution W₀ is supplied from the metal ion-containing solution storage tank 20 through the pipe 50 and a bacterial culture A₀ is supplied from the bacterial culture storage tank 22 through the pipe 52. An electron donor B is supplied from the electron donor storage tank 24 through the pipe 54 in a case in which a metal ion is reduced into a metal fine particle and also the metal fine particle is accumulated in a microorganism.

In the reaction solution storage tank 10, the metal ion-containing solution W₀, the bacterial culture A₀, and the electron donor B which have been supplied are stirred and mixed with the stirring blade 10a. A metal ion in the metal ion-containing solution W₀ is reduced to produce a metal fine particle and also the metal fine particle is accumulated in a microorganism in a case in which the electron donor B is supplied together with the metal ion-containing solution W₀ and the bacterial culture A₀. A metal ion in the metal ion-containing solution W₀ is accumulated by being directly adsorbed on a microorganism in a case in which the electron donor B is not supplied. By virtue of this, a solution W₁ that contains a microorganism having a metal fine particle accumulated therein (hereinafter, referred to as the metal fine particle accumulated microorganism) or a microorganism having a metal ion accumulated therein (hereinafter, referred to as the metal ion accumulated microorganism) is obtained.

As the material of the reaction solution storage tank 10, a known material can be adopted as long as it is hardly corroded by the metal ion-containing solution W₀, the bacterial culture A₀, and the electron donor B and does not adversely affect the action of the microorganism and the electron donor.

### [(b) Concentration unit]

To the membrane module 14 in the concentration unit 3, the solution W₁ containing the metal fine particle accumulated microorganism or the metal accumulated microorganism is supplied from the reaction solution storage tank 10 through the pipe 56.

A secondary side (filtered water side) 14b of the filtration membrane in the membrane module 14 is connected to the filtration pump 48 through the pipe 46. A concentrated solution W₂ containing the metal fine particle accumulated microorganism or the metal ion accumulated microorganism is obtained from a primary side 14a of the filtration membrane and a filtered water W₃ is obtained from the secondary side 14b as the filtration pump 48 works.

The filtration membrane is not particularly limited as long as a membrane can capture the metal fine particle accumulated microorganism or the metal ion accumulated microorganism, and it is possible to use a known membrane such as a hollow fiber membrane, a flat membrane, a tubular membrane and a monolithic film. Among them, a hollow fiber membrane is preferable as the filtration membrane since it has a high volume packing factor.

The material of the filtration membrane is not particularly limited as long as a material is not corroded by the solution W₁ containing the metal fine particle accumulated microorganism or the metal ion accumulated microorganism, and it is possible to use an inorganic material such as ceramics and an organic material such as cellulose, a cellulose mixed ester, a polyolefin, a polysulfone, polyvinylidene fluoride (PVDF) and polytetrafluoroethylene (PTFE). Among them, ceramics, polyvinylidene fluoride (PVDF) and polytetrafluoroethylene (PTFE) are preferable as the material of the filtration membrane from the viewpoint of being strong against a chemical or a pH change.

The average pore size of the micropores formed on the filtration membrane is not particularly limited as long as the metal fine particle accumulated microorganism or the metal ion accumulated microorganism can be captured, and it is preferably from 0.01 to 1.0 µm and more preferably from 0.05 to 0.4 µm.

It is easy to decrease the pressure required for membrane filtration and to increase the concentration efficiency of the metal fine particle or the metal ion when the average pore size of the micropores is equal to or greater than the lower limit value. In addition, it is easy to suppress the leakage of the metal fine particle accumulated microorganism or the metal ion accumulated microorganism to the secondary side of the filtration membrane when the average pore size of the micropores is equal to or less than the upper limit value.

The effective membrane area of the membrane module 14 is not particularly limited, and it may be a membrane area in which a desired amount of the filtered water W₃ is obtained by the filtration pump 48. The effective membrane area of the membrane module 14 is preferably set to a membrane area so as to have a filtration linear velocity (LV) of from 0.1 to 1 m/day when obtaining a desired amount of the filtered water W₃ by the filtration pump 48 from the viewpoint of favorable filtration efficiency and prevention of membrane clogging.

### [(c) Return unit]

In the return unit 4, the concentrated solution W₂ obtained from the primary side 14a of the filtration membrane in the membrane module 14 is returned to the reaction solution storage tank 10 through the pipe 58 and circulated. By virtue of this, the cell concentration of the solution W₁ containing the metal fine particle accumulated microorganism or the metal ion accumulated microorganism in the reaction solution storage tank 10 increases. In addition, it is possible to enhance the concentration efficiency since the microorganism in the concentrated solution W₂ which has been returned is reusable in the reaction solution storage tank 10 unless otherwise the metal fine particle accumulated microorganism or the metal ion accumulated microorganism loses the ability to accumulate the metal fine particle or to adsorb the metal ion.

### [(a₀) Supply unit]

The device for metal recovery 1 of this example includes the supply unit 6 including the metal ion-containing solution storage tank 20, the bacterial culture storage tank 22 and the electron donor storage tank 24.

The metal ion-containing solution storage tank 20 is a storage tank to temporarily store the metal ion-containing solution W₀. The metal ion-containing solution storage tank 20 is not particularly limited as long as a storage tank can store the metal ion-containing solution W₀.

The bacterial culture storage tank 22 is a storage tank to temporarily store the bacterial culture A₀. The bacterial culture storage tank 22 is not particularly limited as long as a storage tank can store the bacterial culture A₀.

The electron donor storage tank 24 is a storage tank to temporarily store the electron donor B which is required when the metal ion contained in the metal ion-containing solution W₀ is reduced into the metal fine particle by the action of the microorganism. The electron donor storage tank 24 is not particularly limited as long as a storage tank can store the electron donor B.

### [(d) Recovery unit]

The device for metal recovery 1 of this example includes the recovery unit 16 to recover the solution W₁ containing the metal fine particle accumulated microorganism or the metal ion accumulated microorganism which is sent from the reaction solution storage tank 10 through the pipe 44.

The recovery unit 16 is not particularly limited as long as a recovery unit can recover the solution W₁ containing the metal fine particle accumulated microorganism or the metal ion accumulated microorganism.

In addition, the device for metal recovery 1 may be a device in which the solution W₁ flows to the recovery unit 16 and the solution W₁ is recovered as the valve and pump provided to the passage (for example, pipe 44) from the reaction solution storage tank 10 to the recovery unit 16 work when the measurement unit 5 to be subsequently described detects that the cell concentration is equal to or greater than a predetermined value.

### [(e) Measurement unit]

The device for metal recovery 1 of this example includes the measurement unit 5 to measure the cell concentration of the solution W₁ containing the metal fine particle accumulated microorganism or the metal ion accumulated microorganism to be supplied from the reaction solution storage tank 10 of the storage unit 2 to the concentration unit 3.

The measurement unit 5 of this example is installed outside the reaction solution storage tank 10 and measures the cell concentration of the solution W₁ containing the metal fine particle accumulated microorganism or the metal ion accumulated microorganism sampled from the reaction solution storage tank 10. Incidentally, the measurement unit 5 may be designed so as to directly measure the cell concentration of the solution W₁ containing the microorganism having a metal fine particle or a metal ion accumulated therein inside the reaction solution storage tank 10 or inside the pipe 56.

The measurement unit 5 is not particularly limited as long as a measurement unit can measure the cell concentration of the solution W₁ containing the microorganism having a metal fine particle or a metal ion accumulated therein.

### [Filtered water storage tank]

The device for metal recovery 1 of this example includes the filtered water storage tank 18 to store the filtered water W₃ that has passed through the filtration membrane of the membrane module 14. The filtered water storage tank 18 is not particularly limited as long as a storage tank can store the filtered water W₃.

The filtered water storage tank 18 may include a pH adjusting means to adjust the pH of the filtered water W₃ to the range that is suitable for discharge into a river, if necessary.

The pH adjusting means may be one which can adjust the pH of the filtered water W₃ to a desired pH, and examples thereof may include a pH meter and a means equipped with an acid addition apparatus and an alkali addition apparatus.

### [Mechanism of action]

In the device for metal recovery 1, the metal ion-containing solution W₀, the bacterial culture A₀ and the electron donor B are supplied from the metal ion-containing solution storage tank 20, the bacterial culture storage tank 22 and the electron donor storage tank 24 to the reaction solution storage tank 10 of the storage unit 2, respectively, in a case in which a metal ion is reduced into a metal fine particle by a microorganism and also the metal fine particle is accumulated in the microorganism. The metal ion-containing solution W₀ and the bacterial culture A₀ are supplied from the metal ion-containing solution storage tank 20 and the bacterial culture storage tank 22 to the reaction solution storage tank 10 of the storage unit 2, respectively, in a case in which a metal ion is directly adsorbed on and accumulated in a microorganism.

In the reaction solution storage tank 10, the metal ion contained in the metal ion-containing solution W₀ is reduced into a metal fine particle by the microorganism and also the metal fine particle is accumulated in the microorganism or the metal ion is directly adsorbed on and accumulated in the microorganism in a reaction solution containing the metal ion-containing solution W₀, the bacterial culture A₀ and, if necessary, the electron donor B. By virtue of this, the solution W₁ containing the metal fine particle accumulated microorganism or the metal ion accumulated microorganism is obtained.

A portion of the solution W₁ containing the metal fine particle accumulated microorganism or the metal ion accumulated microorganism is transferred from the reaction solution storage tank 10 of the storage unit 2 to the concentration unit 3 and filtered through the filtration membrane included in the membrane module 14. The concentrated solution W₂ in which the metal fine particle accumulated microorganism or the metal ion accumulated microorganism is concentrated is returned to the reaction solution storage tank 10 through the pipe 58 of the return unit 4. By virtue of this, the cell concentration of the solution W₁ containing the metal fine particle accumulated microorganism or the metal ion accumulated microorganism in the reaction solution storage tank 10 increases in this manner, and as a result, the concentration of the metal fine particle or metal ion increases.

The solution W₁ containing the metal fine particle accumulated microorganism or the metal ion accumulated microorganism which has been concentrated to a desired cell concentration is recovered to the recovery unit 16.

The filtered water W₃ filtered through the filtration membrane is discharged after being subjected to the pH adjustment in the filtered water storage tank 18 if necessary.

Examples of the device for metal concentration of the invention may include a device which has the same configuration as the device for metal recovery 1 except that the pipe 44 and the recovery unit 16 are not included.

In the device for metal recovery or device for metal concentration of the invention described above, the cell concentration in the storage unit is increased as a portion of the solution containing the metal fine particle accumulated microorganism obtained by reducing the metal ion contained in the metal ion-containing solution into a metal fine particle and also accumulating the metal fine particle or the metal ion accumulated microorganism obtained by directly adsorbing and accumulating the metal ion is transferred to and concentrated in the concentration unit and the concentrated solution is returned to the storage unit. Hence, the metal fine particle accumulated microorganism or the metal ion accumulated microorganism can be reused for the accumulation of the metal ion while being concentrated, thus it is possible to concentrate the metal in the metal ion-containing solution with high efficiency, and as a result, it is possible to recover the metal with high efficiency. In addition, the pH control is also easy since it is not required to convert the metal ion contained in the metal ion-containing solution into a hydroxide in order to recover the metal, and thus the operation is convenient.

Incidentally, the device for metal concentration or the device for metal recovery of the invention is not limited to the device for metal concentration having the same configuration as the device for metal recovery 1 or the device for metal recovery 1.

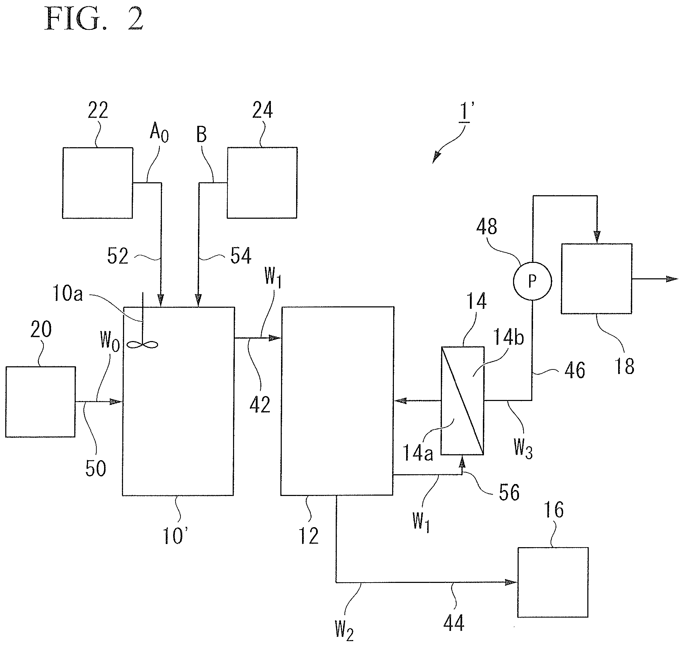

For example, the device for metal concentration or the device for metal recovery of the invention may be a device in which the reaction to reduce a metal ion in a metal ion-containing solution into a metal fine particle by a microorganism and also to accumulate the metal fine particle in the microorganism or the reaction to directly adsorb and accumulate a metal ion in a microorganism is conducted in the pipe (line) of the preceding stage of the reaction solution storage tank by mixing a metal ion-containing solution, an electron donor and a microorganism or a device (device for metal recovery 1') in which the reaction solution storage tank is separately provided as a reaction tank and a concentration tank as illustrated in Fig. 2. In addition, the device for metal concentration or the device for metal recovery of the invention may be a device which does not include an electron donor storage tank in the case of directly adsorbing and accumulating a metal ion in a microorganism, namely, not conducting a reduction reaction of the metal ion.

For example, in the case of using the device for metal recovery 1', in the method for metal recovery using the device for metal recovery 1', the metal ion-containing solution W₀, the bacterial culture A₀ and the electron donor B are supplied to a reaction tank 10' and the microorganism contained in the bacterial culture A₀ is allowed to act on the metal ion-containing solution W₀ so as to reduce the metal ion in the metal ion-containing solution W₀ into a metal fine particle by the microorganism and also to accumulate the metal fine particle in the microorganism in the reaction tank 10'. In addition, the solution W₁ that contains the microorganism having the metal fine particle accumulated therein is transferred to the concentration tank 12 while allowing the microorganism to act on the metal ion-containing solution W₀.

Specifically, while stirring with a stirring blade 10a, to the reaction tank 10', the metal ion-containing solution W₀ is supplied from the metal ion-containing solution storage tank 20 through the pipe 50, the bacterial culture A₀ is supplied from the bacterial culture storage tank 22 through the pipe 52 and the electron donor B is supplied from the electron donor storage tank 24 depending on the metal species. In addition, a portion of the solution W₁ containing the microorganism having the metal ion accumulated therein is continuously withdrawn and transferred to the concentration tank 12 through a pipe 42 while reducing the metal ion in the metal ion-containing solution into a metal fine particle by the microorganism and also accumulating the metal fine particle in the microorganism in the reaction tank 10'.

In addition, the device for metal concentration or the device for metal recovery of the invention may be a device which does not include any one or more of the measurement unit, the supply unit and the filtered water storage tank.

In addition, it may be a device which is equipped with a recovery unit to recover the solution containing the metal fine particle accumulated microorganism or the solution containing the metal ion accumulated microorganism from the concentration unit, or it may be a device which is equipped with a recovery unit to recover the solution containing the metal fine particle accumulated microorganism or the solution containing the metal ion accumulated microorganism from both of the storage unit and the concentration unit.

In addition, in a case in which the electron donor remains in the filtered water, the device for metal concentration or the device for metal recovery of the invention may be a device which is provided, for example, with a membrane that can concentrate the remaining electron donor such as a reverse osmosis membrane and a return pipe to return an aqueous solution containing the concentrated electron donor to the reaction solution storage tank or the electron donor storage tank and thus by which the electron donor remaining in the filtered water is recovered and utilized.

In addition, in a case in which it is required to remove oxygen in the reaction solution upon reducing the metal ion contained in the metal ion-containing solution into the metal fine particle and also accumulating the metal fine particle in the microorganism or adsorbing the metal ion directly on the microorganism, the device for metal concentration or the device for metal recovery of the invention may be a device which includes a means to remove oxygen in at least either of the reaction solution storage tank or the preceding stage of the reaction solution storage tank. Specifically, it may be a device for metal concentration or a device for metal recovery in which, for example, any one or more of the metal ion-containing solution storage tank, the bacterial culture storage tank, the electron donor storage tank and the reaction solution storage tank and the pipe to which the metal ion-containing solution, the bacterial culture or the electron donor is supplied is equipped with a means to remove oxygen.

Examples of the oxygen removing means may include a deaerator and a nitrogen aerator.

### <Method for metal concentration or method for metal recovery>

The method for metal concentration of the invention is a method to concentrate a specific metal in a solution containing a metal ion. The method for metal concentration of the invention is divided into a method to concentrate a metal by utilizing a reducing and accumulating action of a microorganism and a method to concentrate a metal by utilizing the adsorbing action of a microorganism.

In addition, the method for metal recovery of the invention is a method to concentrate and recover a specific metal in a solution containing a metal ion.

Hereinafter, as an example of the method for metal concentration or method for metal recovery of the invention, a method using the device for metal recovery 1 described above will be described.

### [First embodiment: concentration method by reducing and accumulating action of microorganism]

The method for metal concentration using the device for metal recovery 1 of the present embodiment includes the following steps (1) to (3). In addition, the method for metal recovery includes the following step (4) in addition to the following steps (1) to (3).

In the invention, a method which does not include the following step (4) is defined as a method for metal concentration and a method which includes the following step (4) is defined as a method for metal recovery. In other words, to accumulate a metal in a microorganism and to increase the liquid phase cell concentration through separation of the microorganism by a filtration membrane and return thereof is referred to as the "concentration" and to take out a solution that contains the microorganism having the metal accumulated therein to the outside of the system is referred to as the "recovery".
(1) A reduction and accumulation step to reduce the metal ion into a metal fine particle and also to accumulate the metal fine particle in a microorganism by allowing a microorganism and an electron donor to act on a metal ion-containing solution W₀ and thus to obtain a solution W₁ containing a metal fine particle accumulated microorganism.
(2) A concentration step to concentrate the solution W₁ containing a metal fine particle accumulated microorganism by a filtration membrane and thus to obtain a concentrated solution W₂.
(3) A return step to return the concentrated solution W₂ to step (1) above and thus to circulate.
(4) A recovery step to recover the solution W₁ containing a metal fine particle accumulated microorganism.

### (Step (1))

The metal ion-containing solution W₀, the bacterial culture A₀ and the electron donor B are supplied to the reaction solution storage tank 10, respectively, and a microorganism contained in the bacterial culture A₀ and the electron donor B are allowed to act on the metal ion-containing solution W₀ so as to reduce the metal ion in the metal ion-containing solution W₀ into a metal fine particle by the microorganism and also to accumulate the metal fine particle in the microorganism. Specifically, while stirring with the stirring blade 10a, to the reaction solution storage tank 10, the metal ion-containing solution W₀ is supplied from the metal ion-containing solution storage tank 20 through the pipe 50, the bacterial culture A₀ is supplied from the bacterial culture storage tank 22 through the pipe 52 and the electron donor B is supplied from the electron donor storage tank 24 through the pipe 54. In a reaction solution containing the metal ion-containing solution W₀, the bacterial culture A₀ and the electron donor B, the microorganism reduces the metal ion into a metal fine particle and also accumulates the metal fine particle therein and thus the solution W₁ containing the metal fine particle accumulated microorganism is obtained.

The microorganism used in the present embodiment is preferably iron-reducing bacteria.

The iron-reducing bacteria cause the oxidation-reduction reaction using the electron donor B and a metal ion as the electron acceptor in the case of a metal such as Au, Pt and Pd. At this time, the iron-reducing bacteria reduce a metal ion contained in the metal ion-containing solution into a metal fine particle and also accumulate the metal fine particle in their cells. Hence, the use of iron-reducing bacteria makes it possible to concentrate a metal ion in the metal ion-containing solution W₀ as a metal fine particle.

Hence, the present embodiment is especially suitable when concentrating ions of one or more elements selected from the group consisting of Au, Ag, Pt, Pd, Rh, Ir, Ru and Os, the so-called precious metal as a metal fine particle.

Examples of the iron-reducing bacteria may include the following bacteria.

The genus Shewanella such as Shewanella algae (culture collection institute (ATCC): 51181 strain and the like), Shewanella oneidensis (ATCC: 700550 strain and the like).

The genus Geobacter such as Geobacter metallireducens (ATCC: 53774 strain and the like).

The genus Desulfuromonas such as Desulfuromonas palmitatis (ATCC: 51701 strain and the like).

The genus Desulfuromusa such as Desulfuromusa kysingii (DSM (Deutsche Sammlung von Mikroorganismen und Zellkulturen): 7343 strain and the like).

The genus Pelobacter such as Pelobacter venetianus (ATCC: 2394 strain and the like).

The genus Ferrimonas such as Ferrimonas balearica (DSM: 9799 strain and the like).

The genus Aeromonas such as Aeromonas hydrophila (ATCC: 15467 strain and the like).

The genus Sulfurospirillum such as Sulfurospirillum barnesii (ATCC: 700032 strain and the like).

The genus Wolinella such as Wolinella succinogenes (ATCC: 29543 strain and the like).

The genus Desulfovibrio such as Desulfovibrio desulfuricans (ATCC: 29577 strain and the like).

The genus Geothrix such as Geothrix fermentans (ATCC: 700665 strain and the like).

The genus Deferribacter such as Deferribacter thermophiles (DSM: 14813 strain and the like).

The genus Geovibrio such as Geovibrio ferrireducens (ATCC: 51996 strain and the like).

The genus Pyrobaculum such as Pyrobaculum islandicum (DSM: 4184 strain and the like).

The genus Thermotoga such as Thermotoga maritime (DSM: 3109 strain and the like).

The genus Archaeoglobus such as Archaeoglobus fulgidus (ATCC: 49558 strain and the like).

The genus Pyrococcus such as Pyrococcus furiosus (ATCC: 43587 strain and the like).

The genus Pyrodictium such as Pyrodictium abyssi (DSM: 6158 strain and the like).

These iron-reducing bacteria are anaerobic bacteria.

As the iron-reducing bacteria, bacteria belonging to the genus Shewanella are more preferable and Shewanella algae and Shewanella oneidensis are even more preferable from the viewpoint that the accumulation efficiency of a precious metal or a platinum group metal is more favorable.

The microorganism may be one kind or two or more kinds.

The electron donor B is a substance required in a case in which the reduction reaction of the metal ion by an enzyme and the like of the microorganism and the like are accompanied in the case of reducing the metal ion into a metal fine particle by the microorganism and also accumulating the metal fine particle in the microorganism. As the electron donor B, an optimal one may be appropriately used according to the kind of the microorganism.

Examples of the electron donor B may include an electron donor composed of an organic substance having from 1 to 7 carbon atoms and hydrogen gas (molecular hydrogen). Among them, an electron donor composed of an organic substance having from 1 to 7 carbon atoms is preferable as the electron donor B from the viewpoint of ease of handling and solubility in water.

Examples of the electron donor composed of an organic substance having from 1 to 7 carbon atoms may include the following ones.

An aliphatic carboxylic acid salt (fatty acid salt) such as a formic acid salt and an acetic acid salt; an aromatic carboxylic acid salt such as a benzoic acid salt; an oxocarboxylic acid salt such as a pyruvic acid salt; and a carboxylic acid salt such as a lactic acid salt.

An alcohol such as ethanol.

An unsaturated aromatic substance such as toluene phenol.

As the electron donor composed of an organic substance having from 1 to 7 carbon atoms, at least one or both of an aliphatic carboxylic acid having from 1 to 3 carbon atoms and a salt thereof is preferable and at least one or both of formic acid and a salt thereof is more preferable from the viewpoint of solubility in water.

The electron donor B may be one kind or two or more kinds.

The ratio of the amounts of the metal ion-containing solution W₀, the bacterial culture A₀ and the electron donor B supplied to the reaction solution storage tank 10 may be set such that the theoretical initial concentrations thereof in the reaction solution (microorganism cell suspension) in the reaction solution storage tank 10 become a suitable condition in a case in which the metal ion is reduced into a metal fine particle by the microorganism and also the metal fine particle is accumulated in the microorganism. Incidentally, the theoretical initial concentrations refers to the concentrations of the metal ion-containing solution W₀, the bacterial culture A₀ and the electron donor B in the reaction solution in a case in which the reaction has not yet proceeded.

The theoretical initial concentration of the metal ion in the reaction solution in the reaction solution storage tank 10 is preferably from 0.01 to 10 mM and more preferably from 0.1 to 5 mM from the viewpoint that the efficiency to reduce the metal ion into a metal fine particle by the microorganism and also to accumulate the metal fine particle in the microorganism is more favorable.

The theoretical initial concentration of the electron donor B in the reaction solution in the reaction solution storage tank 10 is preferably from 0.2 to 200 mM and more preferably from 1 to 50 mM from the viewpoint that the efficiency to reduce the metal ion into a metal fine particle by the microorganism and also to accumulate the metal fine particle in the microorganism is more favorable.

The theoretical initial concentration of the bacterial culture in the reaction solution in the reaction solution storage tank 10 is preferably from 5.0 × 10¹² to 5.0 × 10¹⁶ cells/m³ and more preferably from 5.0 × 10¹³ to 1.0 × 10¹⁶ cells/m³ from the viewpoint that the efficiency to reduce the metal ion into a metal fine particle by the microorganism and also to accumulate the metal fine particle in the microorganism is more favorable.

In step (1), it is preferable that a metal that is formed by reducing a metal ion coexists when the microorganism and the electron donor act on the metal ion-containing solution from the viewpoint of improving the efficiency of the reduction reaction of the metal ion. The metal is a metal that is obtained by allowing the microorganism and the electron donor used in the present embodiment to act so as to reduce the metal ion into a metal fine particle and also to accumulate the metal fine particle in the microorganism. The metal to coexist is preferably a metal that is obtained by reducing the same metal ion as the metal ion contained in the metal ion-containing solution used in step (1).

The time at which a metal produced by reducing a metal ion is allowed to coexist is not particularly limited, and it is preferable that the metal is supplied and coexists before the metal ion-containing solution W₀ and the electron donor B are supplied or immediately after the supply thereof is started from the viewpoint of further enhancing the efficiency of the reduction reaction.

The coexisting amount of a metal that is produced by reducing a metal ion is not particularly limited and is preferably from 1 to 200% by mass with respect to the produced amount (100% by mass) of the metal obtained by conducting a batch reaction in the reaction solution storage tank 10 in advance.

The form of the metal to coexist is not particularly limited and is preferably a metal fine particle having a particle size of from 0.01 µm to 1 mm since the efficiency of reduction reaction is improved as the surface area is greater. In addition, it is preferable to use the metal fine particle accumulated microorganism obtained by reducing the metal ion in the metal ion-containing solution into a metal fine particle by the microorganism and also accumulating the metal fine particle in the microorganism.

Step (1) may be performed by a batch reaction or by a semi-batch reaction.

In the case of performing step (1) by a semi-batch reaction, it is preferable that the supply of the metal ion-containing solution W₀, the bacterial culture A₀ and the electron donor B to the reaction solution storage tank 10 is adjusted in the range in which the reaction solution in the reaction solution storage tank 10 can be maintained in a predetermined amount in consideration of the amount of the solution W₁ containing the metal fine particle accumulated microorganism which is transferred to the concentration unit 3. The supply of the metal ion-containing solution W₀, the bacterial culture A₀ and the electron donor B to the reaction solution storage tank 10 may be continuous or intermittent as long as the reaction solution in the reaction solution storage tank 10 can be maintained in a predetermined amount.

In the case of performing step (1) by a semi-batch reaction, the average hydraulic retention time (HRT) of the reaction solution in the reaction solution storage tank 10 is preferably from 1 to 120 minutes and more preferably from 5 to 80 minutes. The concentration efficiency of the metal is more favorable when the HRT in the reaction solution storage tank 10 is equal to or longer than the lower limit value, and as a result, the recovery efficiency of the metal is more favorable. The treatment efficiency of the metal ion-containing solution is more favorable when the HRT in the reaction solution storage tank 10 is equal to or shorter than the upper limit value.

The HRT in the reaction solution storage tank 10 can be adjusted by the supply rate of the metal ion-containing solution W₀ to the reaction solution storage tank 10, the transfer rate of the solution W₁ containing the metal fine particle accumulated microorganism from the reaction solution storage tank 10 to the concentration unit 3 and the withdrawal rate of the filtered water W₃ from the membrane module 14.

### (Step (2))

In step (2), a portion of the solution W₁ containing the metal fine particle accumulated microorganism is transferred from the reaction solution storage tank 10 to the membrane module 14 of the concentration unit 3 and concentrated by the filtration membrane to obtain the concentrated solution W₂.

Specifically, the solution W₁ containing the metal fine particle accumulated microorganism which has been transferred from the reaction solution storage tank 10 is filtered through the filtration membrane included in the membrane module 14 by allowing the filtration pump 48 to work so as to be separated into the concentrated solution W₂ and the filtered water W₃.

The filtration linear velocity (LV) is preferably from 0.1 to 1 m/day.

The filtered water W₃ that has been filtered through the filtration membrane is recovered through the pipe 46, stored in the filtered water storage tank 18, and then discharged into a river and the like after adjusting the pH thereof if necessary.

### (Step (3))

In step (3), the concentrated solution W₂ obtained in step (2) is returned to the reaction solution storage tank 10 of step (1) to be circulated. By virtue of this, the cell concentration of the solution W₁ containing the metal fine particle accumulated microorganism in the reaction solution storage tank 10 increases.

### (Step (4))

In the present embodiment, it is preferable to recover the solution W₁ containing the metal fine particle accumulated microorganism of which the cell concentration is increased in the reaction solution storage tank 10 to the recovery unit 16 through the pipe 44.

The cell concentration of the solution W₁ containing the metal fine particle accumulated microorganism which is recovered from the reaction solution storage tank 10 is preferably from 1.0 × 10¹⁵ to 5.0 × 10¹⁷ cells/m³ and more preferably from 5.0 × 10¹⁶ to 1.0 × 10¹⁷ cells/m³. The recovery efficiency of the metal is more favorable when the cell concentration is equal to or greater than the lower limit value. It is easy to continuously favorably maintain the solid-liquid separation by membrane separation as well as the treatment efficiency of the metal ion-containing solution is more favorable when the cell concentration is equal to or less than the upper limit value.

The time at which the solution W₁ containing the metal fine particle accumulated microorganism is recovered can be determined, for example, by measuring the cell concentration of the solution W₁ containing the metal fine particle accumulated microorganism in the reaction solution storage tank 10 by the measurement unit 5.

The application of the metal obtained by the method for metal concentration or method for metal recovery of the present embodiment is not particularly limited. In the present embodiment, the metal fine particle is in a state of being accumulated in the microorganism and thus can be used as a catalyst as it is. In addition, for example, the metal fine particle accumulated microorganism is baked in an electric furnace or the like so as to remove the microorganism and impurities, and then the metal fine particle thus recovered or a separately prepared metal mass (with high purity) may be used.

In the method for metal concentration or method for metal recovery according to the first embodiment of the invention described above, a portion of the solution W₁ containing the metal fine particle accumulated microorganism which is obtained in step (1) is concentrated by membrane filtration to obtain the concentrated solution W₂ in step (2) and the concentrated solution W₂ is returned to step (1) to be circulated in step (3), and thus the concentration of the metal fine particle accumulated microorganism of the solution W₁ containing the metal fine particle accumulated microorganism increases. By virtue of this, it is possible to concentrate the metal in the metal ion-containing solution with high efficiency and thus it is possible to recover the metal with high efficiency. In addition, the pH control is also easy since it is not required to convert the metal ion contained in the metal ion-containing solution into a hydroxide when recovering the metal, and thus the operation is convenient.

In addition, the method for metal concentration or method for metal recovery of the first embodiment of the invention is not limited to the method using the device for metal recovery 1 described above.

For example, it may be a method in which the reaction to reduce a metal ion in a metal ion-containing solution into a metal fine particle by a microorganism and also to accumulate the metal fine particle in the microorganism is conducted in the pipe of the preceding stage of the reaction solution storage tank by mixing a metal ion-containing solution, an electron donor and a microorganism.

In addition, it may be a method using a device (device for metal recovery 1') in which the reaction solution storage tank is separately provided as a reaction tank and a concentration tank as illustrated in Fig. 2, for example.

In addition, in a case in which the electron donor remains in the filtered water, it may be a method in which the remaining electron donor is concentrated, for example, using a membrane that can concentrate the electron donor such as a reverse osmosis membrane, an aqueous solution containing the concentrated electron donor is returned to the reaction solution storage tank or the electron donor storage tank, and the remaining electron donor is recovered and utilized.

In addition, in a case in which it is required to remove oxygen in the reaction solution upon reducing the metal ion contained in the metal ion-containing solution into a metal fine particle by the microorganism and also accumulating the metal fine particle in the microorganism, it may be a method in which the removal of oxygen is conducted in at least either of the reaction solution storage tank or the preceding stage of the reaction solution storage tank.

### [Second embodiment: concentration method by adsorbing action of microorganism]

In the above description, the embodiment using a reducing and accumulating action of a microorganism on a metal fine particle is described, but the invention can also be carried out using an adsorbing action of a microorganism depending on the metal ionic species to be the target of concentration.

Hereinafter, an embodiment using an adsorbing action of a microorganism will be described. However, in the second embodiment, the description on the part which is common to the first embodiment will be omitted and the configuration and the mechanism which are different from those of the first embodiment will be described.

The present embodiment is especially suitable when concentrating and recovering ions of one or more elements selected from the group consisting of Ga, In, Zn, Sn and a lanthanoid.

The method for metal recovery using the device for metal recovery 1 of the present embodiment includes the following steps (1') to (4').
(1') An accumulation step to accumulate a metal ion in a microorganism by allowing a microorganism to act on a metal ion-containing solution W₀ and thus to obtain a solution W₁ containing a metal ion accumulated microorganism.
(2') A concentration step to concentrate the solution W₁ containing a metal ion accumulated microorganism by a filtration membrane and thus to obtain a concentrated solution W₂.
(3') A return step to return the concentrated solution W₂ to step (1') above and thus to circulate.
(4') A recovery step to recover the solution W₁ containing a metal ion accumulated microorganism so as to have a cell concentration of 1.0 × 10¹⁷ cells/m³ or less.

### (Step (1'))

The metal ion is directly, namely, without undergoing the oxidation-reduction reaction adsorbed on the cell surface or inside the cell wall of the microorganism as the microorganism is allowed to act on the metal ion-containing solution W₀ that contains an ion of a metal such as Ga, In, Zn, Sn and a lanthanoid. Hence, the metal ion is adsorbed on and accumulated in the microorganism as a microorganism which can adsorb a metal ion is allowed to act on the metal ion-containing solution W₀, and thus the solution W₁ containing a metal ion accumulated microorganism is obtained.

The difference between the accumulation by the reducing and accumulating action of step (1) of the first embodiment and the accumulation by the adsorbing action of step (1') of the second embodiment is whether the microorganism is allowed to act in a state of being able to cause an oxidation-reduction reaction or not.

In step (1'), the metal ion-containing solution W₀ and the bacterial culture A₀ are supplied to the reaction solution storage tank 10, and the microorganism contained in the bacterial culture A₀ is allowed to act on the metal ion-containing solution W₀ so as to adsorb the metal ion in the metal ion-containing solution W₀ on the microorganism in the reaction solution storage tank 10.

Specifically, while stirring with the stirring blade 10a, to the reaction solution storage tank 10, the metal ion-containing solution W₀ is supplied from the metal ion-containing solution storage tank 20 through the pipe 50 and the bacterial culture A₀ is supplied from the bacterial culture storage tank 22 through the pipe 52. In the reaction solution storage tank 10, the metal ion is adsorbed on and accumulated in the microorganism in the reaction solution containing the metal ion-containing solution W₀ and the bacterial culture A₀ and thus the solution W₁ containing the metal ion accumulated microorganism is obtained.

In step (2') of the second embodiment, it is not required to supply the electron donor B since the metal ion is directly adsorbed on the microorganism.

The microorganism is preferably iron-reducing bacteria as in the first embodiment, and, among the iron-reducing bacteria, the genus Shewanella algae is more preferable and Shewanella algae and Shewanella oneidensis are even more preferable from the viewpoint of favorable adsorption efficiency of the metal ion.

The ratio of the amounts of the metal ion-containing solution W₀ and the bacterial culture A₀ supplied to the reaction solution storage tank 10 may be set such that the theoretical initial concentrations thereof in the reaction solution (microorganism cell suspension) in the reaction solution storage tank 10 become a suitable condition in a case in which the metal ion is directly adsorbed on the microorganism.

The theoretical initial concentration of the metal ion in the reaction solution in the reaction solution storage tank 10 is preferably from 0.01 to 10 mM and more preferably from 0.1 to 5 mM from the viewpoint that the efficiency to adsorb the metal ion on the microorganism is more favorable.

The theoretical initial concentration of the bacterial culture in the reaction solution in the reaction solution storage tank 10 is preferably from 5.0 × 10¹² to 5.0 × 10¹⁶ cells/m³ and more preferably from 5.0 × 10¹³ to 1.0 × 10¹⁶ cells/m³ from the viewpoint that the efficiency to adsorb the metal ion on the microorganism is more favorable.

Step (1') may be performed by a batch reaction or by a semi-batch reaction.

In the case of performing step (1') by a semi-batch reaction, it is preferable that the supply of the metal ion-containing solution W₀ and the bacterial culture A₀ to the reaction solution storage tank 10 is adjusted in the range in which the reaction solution in the reaction solution storage tank 10 can be maintained in a predetermined amount in consideration of the amount of the solution W₁ containing the metal ion accumulated microorganism which is transferred to the concentration unit 3. The supply of the metal ion-containing solution W₀ and the bacterial culture A₀ to the reaction solution storage tank 10 may be continuous or intermittent as long as the reaction solution in the reaction solution storage tank 10 can be maintained in a predetermined amount.

### (Step (2'))

Step (2') can be performed in the same manner as step (2) of the first embodiment.

### (Step (3'))

Step (3') can be performed in the same manner as step (3) of the first embodiment. The cell concentration of the solution W₁ containing the metal accumulated microorganism in the reaction solution storage tank 10 increases as the concentrated solution W₂ obtained in step (2') is returned to the reaction solution storage tank 10 of step (1') to be circulated.

### (Step (4'))

In step (4'), the solution W₁ containing the metal ion accumulated microorganism is recovered from the reaction solution storage tank 10 so as to have a cell concentration of 1.0 × 10¹⁷ cells/m³ or less. It is possible to recover the metal ion by recovering the metal ion accumulated microorganism.

The cell concentration of the solution W₁ containing the metal ion accumulated microorganism which is recovered from the reaction solution storage tank 10 is 1.0 × 10¹⁷ cells/m³ or less, preferably from 1.0 × 10¹⁵ to 1.0 × 10¹⁷ cells/m³ and more preferably from 5.0 × 10¹⁶ to 1.0 × 10¹⁷ cells/m³. The concentration efficiency of metal is more favorable when the cell concentration is equal to or greater than the lower limit value. It is easy to continuously favorably maintain the solid-liquid separation by membrane separation as well as the treatment efficiency of the metal ion-containing solution is more favorable when the cell concentration is equal to or less than the upper limit value.

The application of the recovered metal is not particularly limited. The recovered metal compound may be used after baking the metal ion accumulated microorganism in an electric furnace or the like so as to remove the microorganism and impurities.

In the method for metal concentration according to the second embodiment of the invention described above, a portion of the solution W₁ containing the metal ion accumulated microorganism which is obtained in step (1') is concentrated by membrane filtration in step (2') and returned to step (1') to be circulated in step (3'), and thus it is possible to increase the concentration of the metal accumulated microorganism, namely, the concentration of the metal ion. Hence, it is possible to recover the metal from the metal ion-containing solution with high efficiency. In addition, the pH control is also easy since it is not required to convert the metal ion contained in the metal ion-containing solution into a hydroxide in order to recover the metal, and thus the operation is convenient.

Incidentally, the method for metal recovery of the second embodiment of the invention is not limited to the method using the device for metal recovery 1 described above. For example, it may be a method in which the reaction to directly adsorb a metal ion in a metal ion-containing solution on a microorganism in the reaction solution storage tank is conducted in the pipe of the preceding stage of the reaction solution storage tank by mixing a metal ion-containing solution and a microorganism.

In addition, it may be a method using a device (device for metal recovery 1') in which the reaction solution storage tank is separately provided as a reaction tank and a concentration tank as illustrated in Fig. 2, for example.

For example, in the case of using the device for metal recovery 1', the recovery of the solution containing the microorganism having the metal ion adsorbed thereon can be conducted by the same method as that for the solution containing the microorganism having the metal fine particle accumulated therein in the first embodiment.

In the method for metal recovery using the device for metal recovery 1', the metal ion-containing solution W₀ and the bacterial culture A₀ are supplied to the reaction solution storage tank 10', and the microorganism contained in the bacterial culture A₀ is allowed to act on the metal ion-containing solution W₀ so as to adsorb the metal ion in the metal ion-containing solution W₀ on the microorganism in the reaction solution storage tank 10'. In addition, the solution W₁ that contains the microorganism having the metal ion adsorbed thereon is transferred to the concentration tank 12 while allowing the microorganism to act on the metal ion-containing solution W₀.

Specifically, while stirring with the stirring blade 10a, to the reaction solution storage tank 10', the metal ion-containing solution W₀ is supplied from the metal ion-containing solution storage tank 20 through the pipe 50 and the bacterial culture A₀ is supplied from the bacterial culture storage tank 22 through the pipe 52. In addition, a portion of the solution W₁ that contains the microorganism having the metal ion adsorbed thereon is continuously withdrawn and transferred to the concentration tank 12 through the pipe 42 while conducting the adsorption of the metal ion by the microorganism in the reaction solution storage tank 10'.

In addition, in a case in which it is required to remove oxygen in the solution upon directly adsorbing the metal ion contained in the metal ion-containing solution on the microorganism, it may be a method in which the removal of oxygen is conducted in at least either of the reaction solution storage tank or the preceding stage of the reaction solution storage tank.

### EXAMPLES

Hereinafter, the invention will be described in detail with reference to examples, but the invention is not limited by the following description.

### [Preparation of bacterial culture]

As the microorganism, Shewanella algae (51181 strain) or Shewanella oneidensis (700550 strain) sold by the American Type Culture Collection (ATCC) of a culture collection institute was used. The microorganism was cultured in a Tryptic Soy Broth (TSB) liquid medium while shaking at 30°C and culturing was stopped in the logarithmic growth phase. The cultured cells were collected by centrifugation, the supernatant was then removed, and the collected cells were resuspended in a 50 mM potassium-sodium phosphate buffer solution (pH 7.0). The washing operation to remove the supernatant by subjecting the resuspended cells thus obtained to centrifugation was repeated two times and the washed cells were then resuspended in the buffer, whereby the bacterial culture was prepared.

### [Filtration membrane]

As the filtration membrane of the membrane module 14, a composite membrane (outer diameter: 2.8 mm, nominal pore size: 0.05 µm, manufactured by Mitsubishi Rayon Co., Ltd.) made of a polyvinylidene fluoride (PVDF) resin was used.

As the membrane filter, a film (MF-Millipore, diameter: 47 mm, nominal pore size: 0.05 µm, manufactured by Merck Ltd.) made of a cellulose mixed ester was used.

### [Metal concentration]

The concentration of metal in the solution was measured by ICP atomic emission spectrophotometry. An ICP atomic emission spectrophotometer (Shimadzu Corporation, ICEP-9000) was used as the apparatus for measurement.

### [Cell concentration]

The cell concentration in the solution was measured by a spectrophotometer for ultraviolet and visible region.

### [Example 1]

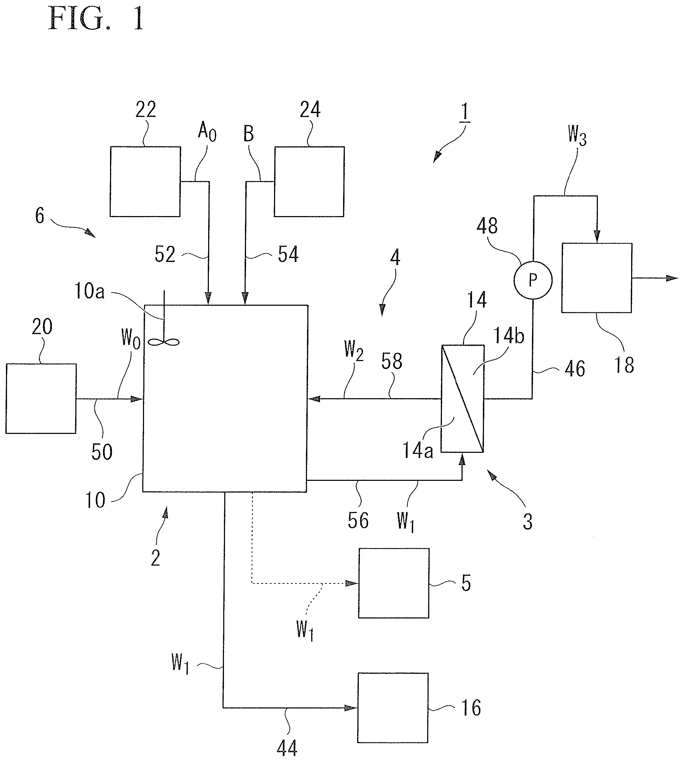

To the reaction solution storage tank 10 of the device for metal recovery 1 illustrated in Fig. 1, 25 mL of an aqueous solution of 10 mM palladium(II) chloride, 20 mL of an aqueous solution of 125 mM sodium formate that was the electron donor and 5 mL of bacterial culture of 1.0 × 10¹⁶ cells/m³ Shewanella algae (S. algae) were supplied such that the theoretical initial concentration of the reaction solution became the following condition, and it was 50 mL in total.
Concentration of Pd(II): 5 mM (530 ppm by mass)
Concentration of sodium formate: 50 mM and
Concentration of cells (S. algae): 1.0 × 10¹⁵ cells/m³

The inside of the reaction solution storage tank 10 was stirred at 30°C and the reduction and accumulation reaction was conducted. After 5 minutes from the start of the reaction, the cells became black as well as the reaction solution which was originally yellow became colorless, and thus the situation was observed that the Pd fine particles were accumulated in the microorganism. After 5 minutes from that moment (after 10 minutes from the start of the reaction), a portion of the solution W₁ containing the Pd fine particle accumulated microorganism was sampled and filtered through a membrane filter having a nominal pore size of 0.05 µm. The concentration of Pd(II) in the filtrate obtained here was measured and the result was about 1 ppm by mass.

Subsequently, a portion the solution W₁ containing the Pd fine particle accumulated microorganism was continuously transferred to the membrane module 14 and separated into the concentrated solution W₂ and the filtered water W₃ by membrane filtration while supplying 150 mL of an aqueous solution of 10 mM palladium(II) chloride per hour and 150 mL of an aqueous solution of 100 mM sodium formate per hour to the reaction solution storage tank 10. The concentrated solution W₂ was returned to the reaction solution storage tank 10 so as to concentrate the Pd fine particle accumulated microorganism in the reaction solution storage tank 10. At this time, the withdrawal rate of the filtered water W₃ was set to 300 mL per hour and the amount of the solution W₁ containing the Pd fine particle accumulated microorganism in the reaction solution storage tank 10 was maintained at 50 mL so that the average hydraulic retention time (HRT) of the reaction solution was 10 minutes. The transfer rate of the solution W₁ from the reaction solution storage tank 10 to the membrane module 14 was 160 L/hr, the filtration linear velocity (LV) was 0.25 m/day, and the return rate of the concentrated solution W₂ from the membrane module 14 to the reaction solution storage tank 10 was 159.7 L/hr.

After 4.5 hours from the start of membrane filtration, the concentration of Pd(II) in the filtered water W₃ was measured and the result was about 1 ppm by mass. From this fact, it was understood that 99% or more of Pd(II) contained in the aqueous solution of 10 mM palladium(II) chloride was accumulated as intracellular fine particles in the reaction solution storage tank 10 and the membrane module 14. In addition, the concentration of Pd(II) in the solution W₁ containing the Pd fine particle accumulated microorganism in the reaction solution storage tank 10 was 140 mM (14840 ppm by mass) and thus the solution W₁ was concentrated to be 14 times the initial aqueous solution of 10 mM palladium(II) chloride. In addition, the cell concentration of the solution W₁ was 1.0 × 10¹⁵ cells/m³. It was understood that 128 g of Pd fine particles per 1 g of dry cells was accumulated since the number of cells per 1 g of dry cells was 8.58 × 10¹².

### [Example 2]

To the reaction solution storage tank 10 of the device for metal recovery 1, 240 mL of an aqueous solution of 1.25 mM palladium(II) chloride, 30 mL of an aqueous solution of 500 mM sodium formate and 30 mL of bacterial culture of 1.0 × 10¹⁶ cells/m³ S. algae were supplied such that the theoretical initial concentration of the reaction solution became the following condition, and it was 300 mL in total.
Concentration of Pd(II): 1 mM (106 ppm by mass)
Concentration of sodium formate: 50 mM and
Concentration of cells (S. algae): 1.0 × 10¹⁵ cells/m³

The inside of the reaction solution storage tank 10 was stirred at 30°C and the reduction and accumulation reaction was conducted. After 30 minutes from the start of the reaction, the cells became black as well as the reaction solution which was originally yellow became colorless, and thus the situation was observed that the Pd fine particles were accumulated in the microorganism. After 30 minutes from that moment (after 1 hour from the start of the reaction), a portion of the solution W₁ containing the Pd fine particle accumulated microorganism was sampled and filtered through a membrane filter having a nominal pore size of 0.05 µm. The concentration of Pd(II) in the filtrate obtained here was measured and the result was about 1 ppm by mass.

Subsequently, a portion the solution W₁ containing the Pd fine particle accumulated microorganism was continuously transferred to the membrane module 14 and separated into the concentrated solution W₂ and the filtered water W₃ by membrane filtration, and the concentrated solution W₂ was returned to the reaction solution storage tank 10 so as to concentrate the Pd fine particle accumulated microorganism in the reaction solution storage tank 10 under the condition presented in Table 1 while supplying an aqueous solution of palladium(II) chloride, an aqueous solution of sodium formate and the bacterial culture of S. algae to the reaction solution storage tank 10 under the supply condition presented in Table 1.

After 9 hours from the start of membrane filtration, the concentration of Pd(II) in the filtered water W₃ was measured and the result was about 1 ppm by mass. From this fact, it was understood that 99% or more of palladium contained in the aqueous solution of 1.25 mM palladium(II) chloride was accumulated as intracellular fine particles in the reaction solution storage tank 10 and the membrane module 14. In addition, the concentration of Pd(II) in the solution W₁ containing the Pd fine particle accumulated microorganism in the reaction solution storage tank 10 was 10 mM (1060 ppm by mass) and thus the solution W₁ was concentrated to be 8 times the aqueous solution of 1.25 mM palladium(II) chloride. In addition, the cell concentration of the solution W₁ was 1.0 × 10¹⁶ cells/m³. It was understood that 0.91 g of Pd fine particles per 1 g of dry cells was accumulated since the number of cells per 1 g of dry cells was 8.58 × 10¹².

### [Example 3]

The pellets of the Pd fine particle accumulated microorganism were obtained by subjecting 50 mL of the solution containing the Pd fine particle accumulated microorganism obtained in Example 1 to centrifugation. The entire amount of the pellets was introduced into the reaction solution storage tank 10 of the device for metal recovery 1 illustrated in Fig. 1, and 40 mL of an aqueous solution of 1.25 mM palladium(II) chloride, 5 mL of an aqueous solution of 500 mM sodium formate and 5 mL of bacterial culture of 1.0 × 10¹⁶ cells/m³ S. algae were then supplied thereto and it was 50 mL in total. The theoretical initial concentration of the reaction solution at this time was as follows.
Concentration of Pd(II): 1 mM (106 ppm by mass)
Concentration of sodium formate: 50 mM
Concentration of cells (S. algae): 1.0 × 10¹⁵ cells/m³
Concentration of Pd fine particle accumulated microorganism: 1.0 × 10¹⁵ cells/m³ and
Concentration of Pd(II) accumulated in microorganism: 140 mM

The inside of the reaction solution storage tank 10 was stirred at 30°C and the reduction and accumulation reaction was conducted. After 5 minutes from the start of the reaction, a portion of the solution W₁ containing the Pd fine particle accumulated microorganism was sampled and filtered through a membrane filter having a nominal pore size of 0.05 µm. The concentration of Pd(II) in the filtrate obtained here was measured and the result was about 1 ppm by mass.

Subsequently, a portion the solution W₁ containing the Pd fine particle accumulated microorganism was continuously transferred to the membrane module 14 and separated into the concentrated solution W₂ and the filtered water W₃ by membrane filtration, and the concentrated solution W₂ was returned to the reaction solution storage tank 10 so as to concentrate the Pd fine particle accumulated microorganism in the reaction solution storage tank 10 under the condition presented in Table 1 while supplying an aqueous solution of palladium(II) chloride, an aqueous solution of sodium formate and the bacterial culture of S. algae to the reaction solution storage tank 10 under the supply condition presented in Table 1.

After 9 hours from the start of membrane filtration, the concentration of Pd(II) in the filtered water W₃ was measured and the result was about 1 ppm by mass. From this fact, it was understood that 99% or more of Pd(II) contained in the aqueous solution of 1.25 mM palladium(II) chloride was accumulated as intracellular fine particles in the reaction solution storage tank 10 and the membrane module 14. The concentration of Pd(II) of the solution W₁ containing the Pd fine particle accumulated microorganism in the reaction solution storage tank 10 was 194 mM. The concentration of Pd(II) accumulated in the microorganism was 54 mM (5724 ppm by mass) when the content of the Pd fine particle accumulated microorganism which was previously introduced was excluded, and thus the solution W₁ was concentrated to be 43.2 times the aqueous solution of 1.25 mM palladium(II) chloride. In addition, the cell concentration of the reaction solution was 5.5 × 10¹⁶ cells/m³ and it was 5.4 × 10¹⁶ cells/m³ when the content of the Pd fine particle accumulated microorganism which was previously introduced was excluded. It was understood that 0.91 g of Pd fine particles per 1 g of dry cells was accumulated when the content of the Pd fine particle accumulated microorganism which was previously introduced was excluded since the number of cells per 1 of dry cells was 8.58 × 10¹².

Thereafter, the supply of the aqueous solution of palladium(II) chloride, the aqueous solution of sodium formate, and the bacterial culture of S. algae was stopped and the membrane filtration and the return of the concentrated solution were continued. After 5 minutes from the stop of the liquid supply, clogging of the membrane occurred, the transmembrane pressure difference (ΔP) was higher than 50 kPa at the time at which the amount of the solution W₁ containing Pd fine particle accumulated microorganism that was contained in the reaction solution storage tank 10 was 25 mL, and thus the operation was stopped. At this time, the cell concentration in the reaction solution storage tank 10 was 1.1 × 10¹⁷ cells/m³.

### [Example 4]

To the reaction solution storage tank 10 of the device for metal recovery 1, 240 mL of an aqueous solution of 1.25 mM platinum(IV) sodium chloride, 30 mL of an aqueous solution of 330 mM sodium lactate and 30 mL of bacterial culture of 1.0 × 10¹⁶ cells/m³ S. algae were supplied such that the theoretical initial concentration of the reaction solution became the following condition, and it was 300 mL in total.
Concentration of Pt(IV): 1 mM (195 ppm by mass)
Concentration of sodium lactate: 33 mM and
Concentration of cells (S. algae): 1.0 × 10¹⁵ cells/m³

The inside of the reaction solution storage tank 10 was stirred at 30°C and the reduction and accumulation reaction was conducted. After 1 hour from the start of the reaction, a portion of the solution W₁ containing the Pt fine particle accumulated microorganism was sampled and filtered through a membrane filter having a nominal pore size of 0.05 µm. The concentration of Pt(IV) in the filtrate obtained here was measured and the result was about 20 ppm by mass.

Subsequently, a portion the solution W₁ containing the Pt fine particle accumulated microorganism was continuously transferred to the membrane module 14 and separated into the concentrated solution W₂ and the filtered water W₃ by membrane filtration, and the concentrated solution W₂ was returned to the reaction solution storage tank 10 so as to concentrate the Pt fine particle accumulated microorganism in the reaction solution storage tank 10 under the condition presented in Table 1 while supplying an aqueous solution of platinum(IV) sodium chloride, an aqueous solution of sodium lactate and the bacterial culture of S. algae to the reaction solution storage tank 10 under the supply condition presented in Table 1.

After 9 hours from the start of membrane filtration, the concentration of Pt(IV) in the filtered water W₃ was measured and the result was about 20 ppm by mass. From this fact, it was understood that about 90% of platinum contained in the aqueous solution of 1.25 mM platinum(IV) sodium chloride was accumulated as intracellular fine particles in the reaction solution storage tank 10 and the membrane module 14. In addition, the concentration of Pt(IV) in the solution W₁ containing the Pt fine particle accumulated microorganism in the reaction solution storage tank 10 was 9 mM (1755 ppm by mass) and thus the solution W₁ was concentrated to be 7.2 times the aqueous solution of 1.25 mM platinum(IV) sodium chloride. In addition, the cell concentration of the solution W₁ was 1.0 × 10¹⁶ cells/m³, and thus it was understood that 1.5 g of Pt fine particles per 1 g of dry cells was accumulated since the number of cells per 1 g of dry cells was 8.58 × 10¹².

### [Example 5]

The reduction and accumulation reaction and concentration of palladium were conducted using the device for metal recovery 1 under the condition presented in Table 2 in the same manner as in Example 2 except that the microorganism was changed to Shewanella oneidensis (S. oneidensis).

After 9 hours from the start of membrane filtration, the concentration of Pd(II) in the filtered water W₃ was measured and the result was about 5 ppm by mass. From this fact, it was understood that about 95% of Pd(II) contained in the aqueous solution of 1.25 mM palladium(II) chloride was accumulated as intracellular fine particles in the reaction solution storage tank 10 and the membrane module 14. In addition, the concentration of Pd(II) in the solution W₁ containing the Pd fine particle accumulated microorganism in the reaction solution storage tank 10 was 9.5 mM (1007 ppm by mass) and thus the solution W₁ was concentrated to be 7.6 times the aqueous solution of 1.25 mM palladium(II) chloride. In addition, the cell concentration of the solution W₁ was 1.0 × 10¹⁶ cells/m³, and thus it was understood was understood that 0.86 g of Pd fine particles per 1 g of dry cells was accumulated since the number of cells per 1 g of dry cells was 8.58 × 10¹².

### [Example 6]

Using the device for metal recovery 1, 346.5 mL of an aqueous solution of 1.1 mM gallium(III) chloride and 38.5 mL of bacterial culture of 7.0 × 10¹⁶ cells/m³ S. algae were supplied such that the theoretical initial concentration of the reaction solution in the reaction solution storage tank 10 became the following condition, and it was 385 mL in total.
Concentration of Ga(III): 1 mM (70 ppm by mass)
Concentration of cells (S. algae): 1.0 × 10¹⁵ cells/m³

The inside of the reaction solution storage tank 10 was stirred at 30°C and the reduction and accumulation reaction was conducted. After 77 minutes from the start of the reaction, a portion of the solution W₁ containing the Ga(III) ion accumulated microorganism was sampled and filtered through a membrane filter having a nominal pore size of 0.05 µm. The concentration of Ga(III) in the filtrate obtained here was measured and the result was about 0.1 ppm by mass.

Subsequently, a portion the solution W₁ containing the Ga(III) ion accumulated microorganism was continuously transferred to the membrane module 14 and separated into the concentrated solution W₂ and the filtered water W₃ by membrane filtration, and the concentrated solution W₂ was returned to the reaction solution storage tank 10 so as to concentrate the Ga(III) ion accumulated microorganism in the reaction solution storage tank 10 under the condition presented in Table 1 while supplying an aqueous solution of gallium(III) chloride and the bacterial culture of S. algae to the reaction solution storage tank 10 under the supply condition presented in Table 2. The transmembrane pressure difference (ΔP) was from 10 to 20 kPa.

After 11.8 hours from the start of membrane filtration, the concentration of Ga(III) in the filtered water W₃ was measured and the result was about 0.1 ppm by mass. From this fact, it was understood that 99% or more of Ga(III) ion contained in the aqueous solution of 1.1 mM gallium(III) chloride was accumulated as intracellular ions in the reaction solution storage tank 10 and the membrane module 14. The concentration of Ga(III) in the solution W₁ containing the Ga(III) ion accumulated microorganism in the reaction solution storage tank 10 was 10 mM (700 ppm by mass) and thus the solution W₁ was concentrated to be 9.1 times the aqueous solution of 1.1 mM gallium(III) chloride. In addition, the cell concentration of the solution W₁ was 7.0 × 10¹⁶ cells/m³, and thus it was understood that 0.085 g of Ga(III) per 1 g of dry cells was accumulated.

Thereafter, the solution W₁ containing the Ga(III) ion accumulated microorganism was recovered from the reaction solution storage tank 10 to the recovery unit 16 by 30 mL per hour. At this time, the supply of the aqueous solution of gallium(III) chloride and the bacterial culture of S. algae, the membrane filtration and the return of the concentrated solution were continued, and the withdrawal rate of the filtered water W₃ was set to 270 mL per hour and the amount of the solution W₂ containing the Ga(III) ion accumulated microorganism that was contained in the reaction solution storage tank 10 was maintained at 385 mL. The transmembrane pressure difference (ΔP) was from 10 to 20 kPa for 10 hours or longer in this state.

Thereafter, the supply of the aqueous solution of gallium(III) chloride and the bacterial culture of S. algae and the recovery to the recover unit 16 were stopped and the membrane filtration and the return of the concentrated solution were continued. After 30 minutes from the stop of the liquid supply, clogging of the membrane occurred, the transmembrane pressure difference (ΔP) was higher than 50 kPa at the time at which the amount of the solution W₁ containing the Ga(III) ion accumulated microorganism that was contained in the reaction solution storage tank 10 was 245 mL, and thus the operation was stopped. At this time, the cell concentration of the reaction solution in the reaction solution storage tank 10 was 1.1 × 10¹⁷ cells/m³.

### [Example 7]

The accumulation and concentration of In(III) were conducted using the device for metal recovery 1 under the conditions presented in Table 2 in the same manner as in Example 6 except that the cells were S. oneidensis and the metal ion-containing solution was 1.1 mM indium(III) chloride.

After 11.8 hours from the start of membrane filtration, the concentration of In(III) in the filtered water W₃ was measured and the result was about 0.1 ppm by mass. From this fact, it was understood that 99% or more of In(III) contained in the aqueous solution of 1.1 mM indium(III) chloride was accumulated as intracellular ions in the reaction solution storage tank 10 and the membrane module 14. The concentration of In(III) in the solution W₁ containing the In(III) ion accumulated microorganism in the reaction solution storage tank 10 was 10 mM (1140 ppm by mass) and thus the solution W₁ was concentrated to be 9.1 times the aqueous solution of 1.1 mM indium(III) chloride. In addition, the cell concentration of the solution W₁ was 7.0 × 10¹⁶ cells/m³, and thus it was understood that 0.14 g of In(III) per 1 g of dry cells was accumulated.

Thereafter, the solution W₁ containing the In(III) ion accumulated microorganism was recovered from the reaction solution storage tank 10 to the recovery unit 16 by 30 mL per hour. At this time, the supply of the aqueous solution of indium(III) chloride and the bacterial culture of S. oneidensis, the membrane filtration and the return of the concentrated solution were continued, and the withdrawal rate of the filtered water W₃ was set to 270 mL per hour and the amount of the solution W₁ containing the In(III) ion accumulated microorganism that was contained in the reaction solution storage tank 10 was maintained at 385 mL. The transmembrane pressure difference (ΔP) was from 10 to 20 kPa for 10 hours or longer in this state.

Thereafter, the supply of the aqueous solution of indium(III) chloride and the bacterial culture of S. oneidensis and the recovery to the recover unit 16 were stopped and the membrane filtration and the return of the concentrated solution were continued. After 30 minutes from the stop of the liquid supply, clogging of the membrane occurred, the transmembrane pressure difference (ΔP) was higher than 50 kPa at the time at which the amount of the solution W₁ containing In(III) ion accumulated microorganism that was contained in the reaction solution storage tank 10 was 245 mL, and thus the operation was stopped. At this time, the cell concentration in the reaction solution storage tank 10 was 1.1 × 10¹⁷ cells/m³.

### [Example 8]

Using the device for metal recovery 1' illustrated in Fig. 2, a Pd(II) containing solution, sodium formate that was the electron donor and the bacterial culture were continuously supplied such that the theoretical initial concentration of the reaction solution (microorganism cell suspension) in the reaction tank 10' became the following condition.
Concentration of Pd(II): 1 mM
Concentration of sodium lactate: 50 mM and
Concentration of cells: 5.0 × 10¹⁵ cells/m³.

The solution W₁ containing the Pd fine particle accumulated microorganism was continuously withdrawn and transferred to the concentration tank 12 so that the average hydraulic retention time (HRT) of the reaction solution in the reaction tank 10' was 60 minutes, the solution W₁ was separated into the concentrated solution W₂ and the filtered water W₃ in the concentration tank 12 by being subjected to membrane filtration by the membrane module 14 so as to concentrate the cells. The filtration linear velocity (LV) for membrane filtration was 0.25 m/day.

The concentration of Pd(II) in the filtered water W₃ was measured, and the ratio of the concentration of Pd(II) in the filtered water W₃ to the concentration of Pd(II) in the reaction tank 10' was determined as the Pd recovery percentage. As a result, 94% or more of Pd(II) introduced into the reaction tank 10' was recovered in the cells. The operating conditions and the results are presented in Table 3.

### [Example 9]

Using the device for metal recovery 1' illustrated in Fig. 2, a Pd(II) containing solution, sodium formate that was the electron donor and the bacterial culture were continuously supplied so that the theoretical initial concentration of the reaction solution (microorganism cell suspension) in the reaction tank 10' became the following condition.
Concentration of Pd(II): 1 mM
Concentration of sodium formate: 200 mM and
Concentration of cells: 1.0 × 10¹⁵ cells/m³

The solution W₁ containing the Pd fine particle accumulated microorganism was continuously withdrawn and transferred to the concentration tank 12 so that the average hydraulic retention time (HRT) of the reaction solution in the reaction tank 10' was 10 minutes, the solution W₁ was separated into the concentrated solution W₂ and the filtered water W₃ in the concentration tank 12 by being subjected to membrane filtration by the filtration membrane so as to concentrate the cells. The filtration linear velocity (LV) for membrane filtration was 0.25 m/day.

The concentration of Pd(II) in the filtered water W₃ was measured, and the ratio of the concentration of Pd(II) in the filtered water W₃ to the concentration of Pd(II) in the reaction tank 10' was determined as the Pd recovery percentage. The concentration of Pd(II) in the filtered water W₃ was measured, but the result was equal to or less than the detection limit (0.1 ppm by mass), and thus 99.8% or more of Pd introduced into the reaction tank 10' was recovered in the cells. The operating conditions and the results are presented in Table 3.

### [Example 10]

Using the device for metal recovery 1' illustrated in Fig. 2, a Ga containing solution and a bacterial culture were continuously supplied so that the theoretical initial concentration of the reaction solution (microorganism cell suspension) in the reaction tank 10' became the following condition.
Concentration of Ga(III): 0.5 mM and
Concentration of cells: 0.7 × 10¹⁶ cells/m³

The solution W₁ that contained the microorganism having Ga(III) ion adsorbed thereon was continuously withdrawn and transferred to the concentration tank 12 so that the average hydraulic retention time (HRT) of the reaction solution in the reaction tank 10' was 77 minutes, the solution W₁ was separated into the concentrated solution W₂ and the filtered water W₃ in the concentration tank 12 by being subjected to membrane filtration by the membrane module 14 so as to concentrate the cells. The filtration linear velocity (LV) for membrane filtration was 0.25 m/day.

The concentration of Ga(III) in the filtered water W₃ was measured, and the ratio of the concentration of Ga(III) in the filtered water W₃ to the concentration of Ga(III) in the reaction tank 10' was determined as the Ga recovery percentage. The concentration of Ga(III) in the filtered water W₃ was measured, but the result was equal to or less than the detection limit (0.1 ppm by mass), and thus 99.8% or more of Ga(III) introduced into the reaction tank 10' was recovered in the cells. The operating conditions and the results are presented in Table 3.

### [Comparative Example 1]

The reduction and accumulation reaction and concentration of palladium were conducted using the device for metal recovery 1 under the conditions presented in Table 3 in the same manner as in Example 1 except that the membrane module 14 was changed to a suction filter using a membrane filter, the transfer rate of the solution to the filter and the withdrawal rate of the filtered water W₃ were set to 300 mL per hour and the Pd fine particle accumulated microorganism to be obtained was not returned to the reaction solution storage tank 10.

After 4.5 hours from the start of membrane filtration, the concentration of Pd(II) in the filtered water W₃ was measured and the result was about 500 ppm by mass. From this fact, it was understood that about 95% of Pd(II) contained in the aqueous solution of 10 mM Palladium(II) chloride was discharged to the outside of the system as the filtered water W₃. The accumulated amount of palladium per 1 g of cells was 10.9 g, but it was considered that most of palladium accumulated in cells was those which were accumulated at the time of the initial reduction and accumulation reaction. It was indicated that the recovery percentage from the solution and the accumulated amount of palladium per 1 g of cells were remarkably decreased as compared with Example 1 in which the Pd fine particle accumulated microorganism was returned.

**[Table 1]**

| | | | | Example 1 | Example 2 | Example 3 | Example 4 |
|---|---|---|---|---|---|---|---|
| Reduction and accumulation step | Species of microorganism | | | S. algae | S. algae | S. algae | S. algae |
| | Initial conditions | Initial concentration of cells [cells/m³] | | 1.0×10¹⁵ | 1.0×10¹⁵ | 1.0×10¹⁵ | 1.1×10¹⁵ |
| | | Initial concentration of metal ion [mM] | | 5 | 1 | 1 | 1 |
| | | Initial concentration of electron donor [mM] | | 50 | 50 | 50 | 33 |
| | Supply conditions | Bacterial culture | Concentration [cells/m³] | - | 1.0×10¹⁶ | 1.0×10¹⁶ | 1.1×10¹⁶ |
| | | | Supply rate [mL/hr] | - | 30 | 30 | 30 |
| | | Metal ion-containing solution | Kind of metal compound | PdCl₂ | PdCl₂ | PdCl₂ | Na₂PtCl₆ |
| | | | Concentration [mM] | 10 | 1.25 | 1.25 | 1.25 |
| | | | Supply rate [mL/hr] | 150 | 240 | 240 | 240 |
| | | Electron donor-containing solution | Kind of electron donor | Na formate | Na formate | Na formate | Na lactate |
| | | | Concentration [mM] | 100 | 500 | 500 | 330 |
| | | | Supply rate [mL/hr] | 150 | 30 | 30 | 30 |
| | Presence or absence of addition of reducing metal | | | Absence | Absence | Presence | Absence |
| | Amount of solution maintained in reaction solution storage tank [mL] | | | 50 | 300 | 50 | 300 |
| | Hydraulic retention time of microorganism cell suspension (HRT)[min] | | | 10 | 60 | 10 | 60 |
| Concentration step | Transfer rate of solution W₁ to concentration unit [L/hr] | | | 160 | 160 | 160 | 160 |
| | Before start of recovery | Withdrawal rate of filtered water [mL/hr] | | 300 | 300 | 300 | 300 |
| | | Filtration linear velocity (LV)[m/day] | | 0.25 | 0.25 | 0.25 | 0.25 |
| | After start of recovery | Withdrawal rate of filtered water [mL/hr] | | - | - | - | - |
| | | Filtration linear velocity (LV)[m/day] | | - | - | - | - |
| Return step | Return rate of concentrated solution W₂ [L/hr] | | | 159.7 | 159.7 | 159.7 | 159.7 |
| Recovery step | Start time of recovery (after start of filtration) [hours] | | | 4.5 | 9 | 9 | 9 |
| | Concentration of metal ion in filtered water W₃ [mM] | | | 0.01 | 0.01 | 0.01 | 0.1 |
| | Recovery percentage of metal [%] | | | > 99 | > 99 | > 99 | 90 |
| | Recovery rate [mL/hr] | | | - | - | - | - |
| | Concentration of cells in solution W₁ at the time of recovery [cells/m³] | | | 1.0×10¹⁵ | 1.0×10¹⁶ | 1.0×10¹⁶ | 1.1×10¹⁶ |
| | Concentration of metal in solution W₁ at the time of recovery [mM] | | | 140 | 10 | 54 | 9 |
| | Accumulated amount of metal per 1 g of dry cells [g] | | | 128 | 0.91 | 0.91 | 1.5 |

**[Table 2]**

| | | | | Example 5 | Example 6 | Example 7 |
|---|---|---|---|---|---|---|
| Reduction and accumulation step | Species of microorganism | | | S. oneidensis | S. algae | S. oneidensis |
| | Initial conditions | Initial concentration of cells [cells/m³] | | 1.0×10¹⁵ | 7.0×10¹⁵ | 7.0×10¹⁵ |
| | | Initial concentration of metal ion [mM] | | 1 | 1 | 1 |
| | | Initial concentration of electron donor [mM] | | 50 | - | - |
| | Supply conditions | Bacterial culture | Concentration [cells/m³] | 1.0×10¹⁶ | 7.0×10¹⁶ | 7.0×10¹⁶ |
| | | | Supply rate [mL/hr] | 30 | 30 | 30 |
| | | Metal ion-containing solution | Kind of metal compound | PdCl₂ | GaCl₃ | InCl₃ |
| | | | Concentration [mM] | 1.25 | 1.1 | 1.1 |
| | | | Supply rate [mL/hr] | 240 | 270 | 270 |
| | | Electron donor-containing solution | Kind of electron donor | Na formate | - | - |
| | | | Concentration [mM] | 500 | - | - |
| | | | Supply rate [mL/hr] | 30 | - | - |
| | Presence or absence of addition of reducing metal | | | Absence | Absence | Absence |
| | Amount of solution in reaction solution storage tank [mL] | | | 300 | 385 | 385 |
| | Hydraulic retention time of microorganism cell suspension (HRT) [min] | | | 60 | 77 | 77 |
| Concentration step | Transfer rate of solution W₁ to concentration unit [L/hr] | | | 160 | 160 | 160 |
| | Before start of recovery | Withdrawal rate of filtered water [mL/hr] | | 300 | 300 | 300 |
| | | Filtration linear velocity (LV)[m/day] | | 0.25 | 0.25 | 0.25 |
| | After start of recovery | Withdrawal rate of filtered water [mL/hr] | | - | 270 | 270 |
| | | Filtration linear velocity (LV)[m/day] | | - | 0.23 | 0.23 |
| Return step | Return rate of concentrated solution W₂ [L/hr] | | | 159.7 | 159.7 | 159.7 |
| Recovery step | Start time of recovery [hours] | | | 9 | 11.8 | 11.8 |
| | Concentration of metal ion in filtered water W₃ [mM] | | | 0.01 | 0.005 | 0.005 |
| | Recovery percentage of metal [%] | | | 95 | > 99 | > 99 |
| | Recovery rate [mL/hr] | | | - | 30 | 10 |
| | Concentration of cells in solution W₁ at the time of recovery [cells/m³] | | | 1.0×10¹⁶ | 7.0×10¹⁶ | 30 |
| | Concentration of metal in solution W₁ at the time of recovery [mM] | | | 9.5 | 10 | 10 |
| | Accumulated amount of metal per 1 g of cells [g] | | | 0.86 | 0.085 | 0.14 |

**[Table 3]**

| | | | | Example 8 | Example 9 | Example 10 | Comparative Example 1 |
|---|---|---|---|---|---|---|---|
| Reduction and accumulation step | Species of microorganism | | | S. algae | S. algae | S. algae | S. algae |
| | Initial conditions | Initial concentration of cells [cells/m³] | | 5.0×10¹⁵ | 1.0×10¹⁵ | 7.0×10¹⁵ | 1.0×10¹⁵ |
| | | Initial concentration of metal ion [mM] | | 1 | 1 | 0.5 | 5 |
| | | Initial concentration of electron donor [mM] | | 50 | 200 | - | 50 |
| | Supply conditions | Bacterial culture | Concentration [cells/m³] | 5.0×10¹⁶ | 1.0×10¹⁶ | 7.0×10¹⁶ | - |
| | | | Supply rate [mL/hr] | 30 | 30 | 30 | - |
| | | Metal ion-containing solution | Kind of metal compound | PdCl₂ | PdCl₂ | GaCl₃ | PdCl₂ |
| | | | Concentration [mM] | 1.25 | 1.25 | 0.55 | 10 |
| | | | Supply rate [mL/hr] | 240 | 240 | 270 | 150 |
| | | Electron donor-containing solution | Kind of electron donor | Na formate | Na formate | - | Na formate |
| | | | Concentration [mM] | 500 | 2000 | - | 100 |
| | | | Supply rate [mL/hr] | 30 | 30 | - | 150 |
| | Presence or absence of addition of reducing metal | | | Absence | Absence | Absence | Absence |
| | Amount of solution maintained in reaction solution storage tank [mL] | | | 300 | 50 | 385 | 50 |
| | Hydraulic retention time of reaction solution (HRT)[min] | | | 60 | 10 | 77 | 10 |
| Concentration step | Transfer rate of solution W₁ to concentration unit [L/hr] | | | 160 | 160 | 160 | 0.3 |
| | Before start of recovery | Withdrawal rate of filtered water [mL/hr] | | 300 | 300 | 300 | 300 |
| | | Filtration linear velocity (LV)[m/day] | | 0.25 | 0.25 | 0.25 | - |
| | After start of recovery | Withdrawal rate of filtered water [mL/hr] | | - | - | 270 | - |
| | | Filtration linear velocity (LV)[m/day] | | - | - | 0.23 | - |
| Return step | Return rate of concentrated solution W₂ [L/hr] | | | 159.7 | 159.7 | 159.7 | - |
| Recovery step | Start time of recovery (after start of filtration) [hours] | | | 9 | 9 | 11.8 | 4.5 |
| | Concentration of metal ion in filtered water W₃ [mM] | | | 0.01 | 0.01 | 0.005 | 4.7 |
| | Recovery percentage of metal [%] | | | > 94 | > 99.8 | > 99.8 | 5 |
| | Recovery rate [mL/hr] | | | - | - | 30 | - |
| | Concentration of cells in solution W₁ at the time of recovery [cells/m³] | | | 5.0×10¹⁶ | 1.0×10¹⁶ | 7.0×10¹⁶ | - |
| | Concentration of metal in solution W₁ at the time of recovery [mM] | | | 10 | 54 | 5 | 0.3 |
| | Accumulated amount of metal per 1 g of dry cells [g] | | | 0.18 | 0.91 | 0.043 | 10.9 |

### EXPLANATIONS OF LETTERS OR NUMERALS

- 1 and 1': device for metal recovery
- 2: storage unit
- 3: concentration unit
- 4: return unit
- 5: measurement unit
- 6: supply unit
- 10: reaction solution storage tank
- 10': reaction tank
- 12: concentration tank
- 14: membrane module
- 16: recovery unit and
- 18: filtered water storage tank
This application is a divisional application of European patent application no. 14 740 625.0 (the "parent application"), also published under no. EP 2 947 163 A1. The following items corresponding to the originally filed claims of the parent application form part of the content of this description as filed.

### ITEMS

1. A method for concentrating a metal in a metal ion-containing solution, the method comprising the following steps (1) to (3):
   (1) a reduction and accumulation step to reduce the metal ion into a metal fine particle and also to accumulate the metal fine particle in the microorganism by allowing the microorganism and an electron donor to act on a metal ion-containing solution and thus to obtain a solution that contains a microorganism having a metal fine particle accumulated therein;
   (2) a concentration step to concentrate the solution that contains the microorganism having a metal fine particle accumulated therein by a filtration membrane and thus to obtain a concentrated solution; and
   (3) a return step to return the concentrated solution to step (1) above and thus to circulate.
2. The method for metal concentration according to item 1, wherein the metal ion is ions of one or more elements selected from the group consisting of Au, Ag, Pt, Pd, Rh, Ir, Ru and Os.
3. The method for metal concentration according to item 1 or 2, wherein a metal that is produced by reducing the metal ion coexists when a microorganism and an electron donor act on a metal ion-containing solution in step (1) above.
4. The method for metal concentration according to any one of items 1 to 3, wherein the electron donor is an organic substance having from 1 to 7 carbon atoms.
5. The method for metal concentration according to any one of items 1 to 4, wherein the electron donor is at least one or both of an aliphatic carboxylic acid having from 1 to 3 carbon atoms and a salt thereof.
6. The method for metal concentration according to any one of items 1 to 5, wherein the electron donor is at least one or both of formic acid and a salt thereof.
7. The method for metal concentration according to any one of items 1 to 6, wherein the microorganism is iron-reducing bacteria.
8. The method for metal concentration according to item 7, wherein the iron-reducing bacteria are bacteria belonging to the genus Shewanella.
9. The method for metal concentration according to item 8, wherein the bacteria belonging to the genus Shewanella are Shewanella algae or Shewanella oneidensis.
10. The method for metal concentration according to any one of items 1 to 9, wherein an average pore size of the filtration membrane is from 0.01 to 1.0 µm.
11. A method for metal recovery, the method for recovering the microorganism that is obtained in the method for metal concentration according to any one of items 1 to 10 and has a metal fine particle accumulated therein, the method comprising the following step (4):
   (4) a recovery step to recover the solution that contains a microorganism having the metal fine particle accumulated therein so as to have a cell concentration of 1.0 × 10¹⁷ cells/m³ or less.
12. A method for metal recovery from a metal ion-containing solution, the method comprising the following steps (1') to (4'):
   (1') an accumulation step to accumulate a metal ion in a microorganism by allowing the microorganism to act on a metal ion-containing solution and thus to obtain a solution that contains a microorganism having the metal ion accumulated therein;
   (2') a concentration step to concentrate the solution that contains the microorganism having the metal ion accumulated therein by a filtration membrane and thus to obtain a concentrated solution;
   (3') a return step to return the concentrated solution to step (1') above and thus to circulate; and
   (4') a recovery step to recover the solution that contains the microorganism having a metal fine particle accumulated therein so as to have a cell concentration of 1.0 × 10¹⁷ cells/m³ or less.
13. The method for metal recovery according to item 12, wherein the metal ion is ions of one or more elements selected from the group consisting of Ga, In, Zn, Sn and a lanthanoid.
14. The method for metal recovery according to item 12 or 13, wherein the microorganism is iron-reducing bacteria.
15. The method for metal recovery according to item 14, wherein the iron-reducing bacteria are bacteria belonging to the genus Shewanella.
16. The method for metal recovery according to item 15, wherein the bacteria belonging to the genus Shewanella are Shewanella algae or Shewanella oneidensis.
17. The method for metal recovery according to any one of items 12 to 16, wherein an average pore size of the filtration membrane is from 0.01 to 1.0 µm.
18. A device for metal concentration, comprising the following (a) to (c):
   (a) a storage unit to store a solution that is obtained by allowing a microorganism and an electron donor to act on a metal ion-containing solution so as to reduce a metal ion into a metal fine particle and also to accumulate the metal fine particle in the microorganism or by allowing the microorganism to act on a metal ion-containing solution so as to accumulate a metal ion in the microorganism and contains a microorganism having a metal fine particle or a metal ion accumulated therein;
   (b) a concentration unit to concentrate the solution that is transferred from the storage unit and contains the microorganism having a metal fine particle or a metal ion accumulated therein by a filtration membrane; and
   (c) a return unit to return a concentrated solution that is concentrated in the concentration unit to the storage unit.
19. The device for metal concentration according to item 18, the device further comprising the following (e):
   (e) a measurement unit to measure a cell concentration of the solution that is supplied to the concentration unit and contains the microorganism having a metal fine particle or a metal ion accumulated therein.
20. A device for metal recovery, comprising the following (a) to (d):
   (a) a storage unit to store a solution that is obtained by allowing a microorganism and an electron donor to act on a metal ion-containing solution so as to reduce a metal ion into a metal fine particle and also to accumulate the metal fine particle in the microorganism or by allowing the microorganism to act on a metal ion-containing solution so as to accumulate the metal ion in the microorganism and contains a microorganism having a metal fine particle or the metal ion accumulated therein;
   (b) a concentration unit to concentrate the solution that is transferred from the storage unit and contains the microorganism having a metal fine particle or a metal ion accumulated therein by a filtration membrane;
   (c) a return unit to return a concentrated solution that is concentrated in the concentration unit to the storage unit; and
   (d) a recovery unit to recover the solution that contains the microorganism having a metal fine particle or a metal ion accumulated therein from at least one or both of the storage unit and the concentration unit.
21. The device for metal recovery according to item 20, the device further comprising the following (e):
   (e) a measurement unit to measure a cell concentration of the solution that is supplied to the concentration unit and contains a microorganism having a metal fine particle or a metal ion accumulated therein.

## Claims

1. A method for metal recovery from a metal ion-containing solution, the method comprising the following steps (1') to (4'):
(1') an accumulation step to accumulate a metal ion in a microorganism by allowing the microorganism to act on a metal ion-containing solution and thus to obtain a solution that contains a microorganism having the metal ion accumulated therein;
(2') a concentration step to concentrate the solution that contains the microorganism having the metal ion accumulated therein by a filtration membrane and thus to obtain a concentrated solution;
(3') a return step to return the concentrated solution to step (1') above
and thus to circulate; and
(4') a recovery step to recover the solution that contains the microorganism having a metal fine particle accumulated therein so as to have a cell concentration of 1.0 × 10¹⁷ cells/m³ or less.

2. The method for metal recovery according to claim 1, wherein the metal ion is ions of one or more elements selected from the group consisting of Ga, In, Zn, Sn and a lanthanoid.

3. The method for metal recovery according to claim 1 or 2, wherein the microorganism is iron-reducing bacteria.

4. The method for metal recovery according to claim 3, wherein the iron-reducing bacteria are bacteria belonging to the genus Shewanella.

5. The method for metal recovery according to claim 4, wherein the bacteria belonging to the genus Shewanella are Shewanella algae or Shewanella oneidensis.

6. The method for metal recovery according to any one of claims 1 to 5, wherein an average pore size of the filtration membrane is from 0.01 to 1.0 µm.

7. A device for metal recovery, comprising the following (a) to (d):
(a) a storage unit to store a solution that is obtained by allowing a microorganism and an electron donor to act on a metal ion-containing solution so as to reduce a metal ion into a metal fine particle and also to accumulate the metal fine particle in the microorganism or by allowing the microorganism to act on a metal ion-containing solution so as to accumulate the metal ion in the microorganism and contains a microorganism having a metal fine particle or the metal ion accumulated therein;
(b) a concentration unit to concentrate the solution that is transferred from the storage unit and contains the microorganism having a metal fine particle or a metal ion accumulated therein by a filtration membrane;
(c) a return unit to return a concentrated solution that is concentrated in the concentration unit to the storage unit; and
(d) a recovery unit to recover the solution that contains the microorganism having a metal fine particle or a metal ion accumulated therein from at least one or both of the storage unit and the concentration unit.

8. The device for metal recovery according to claim 7, the device further comprising the following (e):
(e) a measurement unit to measure a cell concentration of the solution that is supplied to the concentration unit and contains a microorganism having a metal fine particle or a metal ion accumulated therein.
